(19)

Europäisches Patentamt
European Patent Office
Office européen des brevets

(11) **EP 2 370 096 B1**

(12) **EUROPEAN PATENT SPECIFICATION**

(45) Date of publication and mention
of the grant of the patent:
**31.05.2017 Bulletin 2017/22**

(51) Int Cl.:
**A61K 39/395** *(2006.01)*     **A61K 31/7068** *(2006.01)*
**A61P 35/00** *(2006.01)*

(21) Application number: **09775304.0**

(86) International application number:
**PCT/IB2009/055391**

(22) Date of filing: **27.11.2009**

(87) International publication number:
**WO 2010/061359 (03.06.2010 Gazette 2010/22)**

(54) **ANTITUMOR COMBINATIONS CONTAINING CYTARABINE AND ANTIBODIES RECOGNIZING SPECIFICALLY CD38**

ANTITUMORKOMBINATIONEN ENTHALTEND CYTARABINE UND ANTIKÖRPER, DIE INSBESONDERE CD38 ERKENNEN

COMBINAISONS ANTITUMORALES CONTENANT CYTARABINE ET DES ANTICORPS RECONNAISSANT SPÉCIFICAMENT CD38

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **28.11.2008 EP 08291118**

(43) Date of publication of application:
**05.10.2011 Bulletin 2011/40**

(73) Proprietor: **SANOFI**
**75008 Paris (FR)**

(72) Inventors:
• **LEJEUNE, Pascale**
**F-75013 Paris (FR)**
• **VRIGNAUD, Patricia**
**F-75013 Paris (FR)**

(74) Representative: **Lavoix**
**2, place d'Estienne d'Orves**
**75441 Paris Cedex 09 (FR)**

(56) References cited:
**EP-A- 1 914 242         WO-A-99/62526**

WO-A-2006/099875

• ELLIS JONATHAN H ET AL: "Engineered anti-CD38 monoclonal antibodies for immunotherapy of multiple myeloma" JOURNAL OF IMMUNOLOGY, AMERICAN ASSOCIATION OF IMMUNOLOGISTS, US, vol. 155, no. 2, 1 January 1995 (1995-01-01), pages 925-937, XP002146232 ISSN: 0022-1767
• PEIPP M ET AL: "FULLY HUMAN CD38 ANTIBODIES EFFICIENTLY TRIGGER ADCC AND CDC OF MULTIPLE MYELOMA AND PLASMA CELL LEUKEMIA CELLS" INTERNET CITATION, [Online] XP002473215 Retrieved from the Internet: URL:http://www.genmab.com/upload/poster_ash_2005_monday_05-12-2005.pdf> [retrieved on 2008-03-18]
• PEIPP M ET AL: "FULLY HUMAN CD38 ANTIBODIES EFFICIENTLY TRIGGER ADCC OF MULTIPLE MYELOMA CELL LINES AND PRIMARY TUMOR CELLS" BLOOD, AMERICAN SOCIETY OF HEMATOLOGY, US, vol. 106, no. 11, PART 01, 1 November 2005 (2005-11-01), page 944A, ABSTRACTNO.3377, XP009069301 ISSN: 0006-4971 & PEIPP M ET AL: "Fully human CD38 antibodies efficiently trigger ADCC of multiple myeloma cell lines and primary tumor cells" BIOSIS,, 1 January 1900 (1900-01-01), XP002389878

**Description**

The present invention is described in claims 1-19 and relates to combinations of monoclonal antibodies directed against CD38 and cytarabine which are therapeutically useful in the treatment of neoplastic diseases.

CD38 is a 45 kD type II transmembrane glycoprotein with a long C-terminal extracellular domain and a short N-terminal cytoplasmic domain. The CD38 protein is a bifunctional ectoenzyme that can catalyze the conversion of NAD$^+$ into cyclic ADP-ribose (cADPR) and also hydrolyze cADPR into ADP-ribose. CD38 is upregulated and has been implicated in many hematopoietic malignancies.

Monoclonal antibodies 38SB13, 38SB18, 38SB19, 38SB30, 38SB31, and 38SB39, which specifically recognize CD38, are described in PCT application WO2008/047242. Said anti-CD38 antibodies are capable of killing CD38$^+$ cells by three different cytotoxic mechanisms, induction of apoptosis, antibody-dependent cell-mediated cytotoxicity (ADCC), and complement-dependent cytotoxicity (CDC). In addition, these antibodies are able to directly induce apoptosis of CD38$^+$ cells, even without the presence of stroma cells or stroma-derived cytokines. Cytarabine is an anti-metabolic agent used in chemotherapy. There is still a need for novel and efficacious medicaments for treating cancer.

It has now been found, and for this invention, that the efficacy of the humanized anti-CD38 antibodies may be considerably improved when it is administered in combination with at least one substance which is therapeutically useful in anticancer treatments and has a mechanism identical to or different from the one of the humanized anti-CD38 antibodies and which is limited in the present invention to cytarabine.

The term "antibody" is used herein in the broadest sense and specifically covers monoclonal antibodies (including full length monoclonal antibodies) of any isotype such as IgG, IgM, IgA, IgD and IgE, polyclonal antibodies, multispecific antibodies, chimeric antibodies, and antibody fragments. A typical IgG antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are usually referred to as CDR1, CDR2, and CDR3, numbered sequentially from the N-terminus. The more highly conserved portions of the variable regions outside of the CDRs are called the "framework regions".

As used herein, "V$_H$" or "VH" refers to the variable region of an immunoglobulin heavy chain of an antibody, including the heavy chain of an Fv, scFv, dsFv, Fab, Fab' or F(ab')2 fragment. Reference to "V$_L$" or "VL" refers to the variable region of the immunoglobulin light chain of an antibody, including the light chain of an Fv, scFv, dsFv, Fab, Fab' or F(ab')2 fragment.

The 38SB13 antibody comprises at least one heavy chain having an amino acid sequence consisting of SEQ ID NO: 50 and at least one light chain having an amino acid sequence consisting of SEQ ID NO: 38, said heavy chain comprising three sequential CDRs having amino acid sequences consisting of SEQ ID NOS: 1, 2, and 3, and said light chain comprising three sequential CDRs having amino acid sequences consisting of SEQ ID NOS: 4, 5, and 6.

The 38SB18 antibody comprises at least one heavy chain having an amino acid sequence consisting of SEQ ID NO: 52 and at least one light chain having an amino acid sequence consisting of SEQ ID NO: 40, said heavy chain comprising three sequential CDRs having amino acid sequences consisting of SEQ ID NOS: 7, 8, and 9, and said light chain comprising three sequential CDRs having amino acid sequences consisting of SEQ ID NOS: 10, 11, and 12.

The 38SB19 antibody comprises at least one heavy chain having an amino acid sequence consisting of SEQ ID NO: 54 and at least one light chain having an amino acid sequence consisting of SEQ ID NO: 42, said heavy chain comprising three sequential CDRs having amino acid sequences consisting of SEQ ID NOS: 13, 14, and 15, and said light chain comprising three sequential CDRs having amino acid sequences consisting of SEQ ID NOS: 16, 17, and 18.

The 38SB30 antibody comprises at least one heavy chain having an amino acid sequence consisting of SEQ ID NO: 56 and at least one light chain having an amino acid sequence consisting of SEQ ID NO: 44, said heavy chain comprising three sequential CDRs having amino acid sequences consisting of SEQ ID NOS: 19, 20, and 21, and said light chain comprising three sequential CDRs having amino acid sequences consisting of SEQ ID NOS: 22, 23, and 24.

The 38SB31 antibody comprises at least one heavy chain having an amino acid sequence consisting of SEQ ID NO: 58 and at least one light chain having an amino acid sequence consisting of SEQ ID NO: 46, said heavy chain comprising three sequential CDRs having amino acid sequences consisting of SEQ ID NOS: 25, 26, and 27, and said light chain comprising three sequential CDRs having amino acid sequences consisting of SEQ ID NOS: 28, 29, and 30.

The 38SB39 antibody comprises at least one heavy chain having an amino acid sequence consisting of SEQ ID NO: 60 and at least one light chain having an amino acid sequence consisting of SEQ ID NO: 48, said heavy chain comprising three sequential CDRs having amino acid sequences consisting of SEQ ID NOS: 31, 32, and 33, and said light chain comprising three sequential CDRs having amino acid sequences consisting of SEQ ID NOS: 34, 35, and 36.

The hybridoma cell lines producing the 38SB13, 38SB18, 38SB19, 38SB30, 38SB31, and 38SB39 murine anti-CD38 antibodies have been deposited at the American Type Culture Collection (10801 University Bid, Manassas, VA, 20110-2209, USA), on June 21, 2006, under the deposit numbers PTA-7667, PTA-7669, PTA-7670, PTA-7666, PTA-7668, and PTA-7671, respectively (as described in WO 2008/047242).

The term "humanized antibody", as used herein, refers to a chimeric antibody which contain minimal sequence derived from non-human immunoglobulin. The goal of humanization is a reduction in the immunogenicity of a xenogenic antibody, such as a murine antibody, for introduction into a human, while maintaining the full antigen binding affinity and specificity of the antibody. Humanized antibodies, or antibodies adapted for non-rejection by other mammals, may be produced using several technologies such as resurfacing and CDR grafting. As used herein, the resurfacing technology uses a combination of molecular modelling, statistical analysis and mutagenesis to alter the non-CDR surfaces of antibody variable regions to resemble the surfaces of known antibodies of the target host. The CDR grafting technology involves substituting the complementarity determining regions of, for example, a mouse antibody, into a human framework domain, e.g., see WO 92/22653. Humanized chimeric antibodies preferably have constant regions and variable regions other than the complementarity determining regions (CDRs) derived substantially or exclusively from the corresponding human antibody regions and CDRs derived substantially or exclusively from a mammal other than a human.

Strategies and methods for the resurfacing of antibodies, and other methods for reducing immunogenicity of antibodies within a different host, are disclosed in US Patent 5,639,641, which is hereby incorporated in its entirety by reference. Antibodies can be humanized using a variety of other techniques including CDR-grafting (EP 0 239 400; WO 91/09967; U.S. Pat. Nos. 5,530,101; and 5,585,089), veneering or resurfacing (EP 0 592 106; EP 0 519 596; Padlan E. A., 1991, Molecular Immunology 28(4/5): 489-498; Studnicka G. M. et al., 1994, Protein Engineering, 7(6): 805-814; Roguska M.A. et al., 1994, PNAS, 91: 969-973), chain shuffling (U.S. Pat. No. 5,565,332), and identification of flexible residues (PCT/US2008/074381). Human antibodies can be made by a variety of methods known in the art including phage display methods. See also U.S. Pat. Nos. 4,444,887, 4,716,111, 5,545,806, and 5,814,318; and international patent application publication numbers WO 98/46645, WO 98/50433, WO 98/24893, WO 98/16654, WO 96/34096, WO 96/33735, and WO 91/10741 (said references incorporated by reference in their entireties).

The anti-CD38 antibodies of the pharmaceutical combination of the present invention are humanized antibodies which recognize CD38 and kill CD38$^+$ cells by apoptosis, ADCC, and CDC. In a further embodiment, the humanized antibodies of the invention are capable of killing said CD38$^+$ cells by apoptosis even in the absence of stroma cells or stroma-derived cytokines.

A preferred embodiment of such a humanized antibody is a humanized 38SB13, 38SB18, 38SB19, 38SB30, 38SB31, or 38SB39 antibody, or an epitope-binding fragment thereof.

The CDRs of the 38SB13, 38SB18, 38SB19, 38SB30, 38SB31, and 38SB39 antibodies are identified by modelling and their molecular structures have been predicted. Thus, in one embodiment, this invention provides humanized antibodies or epitope-binding fragment thereof comprising one or more CDRs having an amino acid sequence selected from the group consisting of SEQ ID NOS: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, and 36. In a preferred embodiment, a humanized version of 38SB13 is provided, which comprises at least one heavy chain and at least one light chain, wherein said heavy chain comprises three sequential complementarity-determining regions having amino acid sequences represented by SEQ ID NOS: 1, 2, and 3, and wherein said light chain comprises three sequential complementarity-determining regions having amino acid sequences represented by SEQ ID NOS: 4, 5, and 6. In another preferred embodiment, a humanized version of 38SB18 is provided, which comprises at least one heavy chain and at least one light chain, wherein said heavy chain comprises three sequential complementarity-determining regions having amino acid sequences represented by SEQ ID NOS: 7, 8, and 9, and wherein said light chain comprises three sequential complementarity-determining regions having amino acid sequences represented by SEQ ID NOS: 10, 11, and 12. In another preferred embodiment, a humanized version of 38SB19 is provided, which comprises at least one heavy chain and at least one light chain, wherein said heavy chain comprises three sequential complementarity-determining regions having amino acid sequences represented by SEQ ID NOS: 13, 14, and 15, and wherein said light chain comprises three sequential complementarity-determining regions having amino acid sequences represented by SEQ ID NOS: 16, 17, and 18. In another preferred embodiment, a humanized version of 38SB30 is provided, which comprises at least one heavy chain and at least one light chain, wherein said heavy chain comprises three sequential complementarity-determining regions having amino acid sequences represented by SEQ ID NOS: 19, 20, and 21, and wherein said light chain comprises three sequential complementarity-determining regions having amino acid sequences represented by SEQ ID NOS: 22, 23, and 24. In another preferred embodiment, a humanized version of 38SB31 is provided, which comprises at least one heavy chain and at least one light chain, wherein said heavy chain comprises three sequential complementarity-determining regions having amino acid sequences represented by SEQ ID NOS: 25, 26, and 27, and wherein said light chain comprises three sequential complementarity-determining regions having amino acid sequences represented by SEQ ID NOS: 28, 29, and 30. In another preferred embodiment, a humanized version of 38SB39 is provided, which comprises at least one heavy chain and at least one light chain, wherein said heavy chain comprises three sequential complementarity-determining regions having amino acid sequences represented by SEQ ID NOS: 31, 32, and 33, and wherein said light chain comprises three sequential complementarity-determining regions having amino acid sequences represented by SEQ ID NOS: 34, 35, and 36.

In one embodiment, this invention provides humanized antibodies or fragments thereof which comprise a V$_H$

having an amino acid sequence selected from the group of SEQ ID NOS: 66 and 72. In a preferred embodiment, a humanized 38SB19 antibody is provided which comprises a $V_H$ having an amino acid sequence represented by SEQ ID NO: 66. In another preferred embodiment, a humanized 38SB31 antibody is provided which comprises a $V_H$ having an amino acid sequence represented by SEQ ID NO: 72.

In another embodiment, this invention provides humanized antibodies or fragments thereof which comprise a $V_L$ having an amino acid sequence selected from the group of SEQ ID NOS: 62, 64, 68, and 70. In a preferred embodiment, a humanized 38SB19 antibody is provided which comprises a $V_L$ having an amino acid sequence chosen from the group of SEQ ID NOS: 62 and 64. In another preferred embodiment, a humanized 38SB31 antibody is provided which comprises a $V_L$ having an amino acid sequence chosen from the group of SEQ ID NOS: 68 and 70.

Each of the humanized versions of the 38SB13, 38SB18, 38SB19, 38SB30, 38SB31, and 38SB39 antibodies has been shown to be particularly advantageous as an anticancer agent. The preparation, physical properties and beneficial pharmacological properties thereof are described in WO 2008/047242. Generally, the doses used for treating human beings, which depend on factors distinctive to the subject to be treated, are between 1 and 150 mg/kg administered orally or between 1 and 150 mg/kg administered intravenously. Cytosine arabinoside or cytarabine or araC (brand name: Aracytin™) is an antimetabolic agent (1β-arabinofuranosylcytosine). Cytarabine is rapidly converted into cytosine arabinoside triphosphosphate, which damages DNA when the cell cycle is in the S phase (synthesis of DNA). Rapidly dividing cells, which require DNA replication for mitosis, are therefore most affected. Cytarabine also inhibits both DNA and RNA polymerases and nucleotide reductase enzymes needed for DNA synthesis. Cytarabine is rapidly deaminated in the body into the inactive uracil derivative and therefore is often given by continuous intravenous infusion.

One aspect of the invention is a pharmaceutical composition comprising an anti-CD38 antibody in combination with at least cytarabine. Since the activity of the products depends on the doses used, it is thus possible to use lower doses and to increase the activity while decreasing the toxicity phenomena. The improved efficacy of a combination according to the invention may be demonstrated by determination of the therapeutic synergy. A combination manifests therapeutic synergy if it is therapeutically superior to the best agent of the study used alone at its maximum tolerated dose or at its highest dose tested when toxicity cannot be reached in the animal species.

This efficacy may be quantified, for example, by the $\log_{10}$ cell kill, which is determined according to the following formula:

$$\log_{10} \text{ cell kill} = \text{T-C (days)}/3.32 \times T_d$$

in which T - C represents the tumor growth delay, which is the median time in days for the tumors of the treated group (T) and the tumors of the control group (C) to reach a predetermined value (1 g for example), and $T_d$ represents the time in days needed for the volume of the tumor to double in the control animals [T.H. Corbett et al., Cancer, 40: 2660-2680 (1977); F.M. Schabel et al., Cancer Drug Development, Part B, Methods in Cancer Research, 17: 3-51, New York, Academic Press Inc. (1979)]. A product is considered to be active if $\log_{10}$ cell kill is greater than or equal to 0.7. A product is considered to be very active if $\log_{10}$ cell kill is greater than 2.8.

The combination, in which each of the constituents will be present at a dose generally not exceeding its maximum tolerated dose, will manifest therapeutic synergy when the $\log_{10}$ cell kill is greater than the value of the $\log_{10}$ cell kill of the best constituent when it is administered alone and used at its maximum tolerated dose or at its highest dose tested.

The efficacy of the combinations on solid tumors may be determined experimentally in the following manner:

The animals subjected to the experiment, generally mice, are subcutaneously grafted bilaterally with 30 to 60 mg of a tumor fragment on day 0. The animals bearing tumors are randomized based on their tumor size before being subjected to the various treatments and controls. Chemotherapy begins when tumors have reached a predetermined size after grafting, depending on the type of tumor, and the animals are observed every day. The different animal groups are weighed daily during treatment until the maximum weight loss is reached and subsequent full weight recovery has occurred. The groups are then weighed once or twice a week until the end of the trial.

The tumors are measured 1 to 5 times a week, depending on the tumor doubling time, until the tumor reaches approximately 2 g, or until the animal dies (if this occurs before the tumor reaches 2 g). The animals are necropsied immediately after euthanasia or death.

The antitumor activity is determined in accordance with the different parameters recorded.

Results obtained with combinations of hu38SB19 and cytarabine used at their optimal doses are indicated hereunder as examples.

The present invention also relates, therefore, to pharmaceutical compositions containing the combinations according to the invention.

The constituents of which the combination are composed may be administered simultaneously, semi-simultaneously, separately, or spaced out over a period of time so as to obtain the maximum efficacy of the combination; it being possible for each administration to vary in its duration from a rapid administration to a continuous perfusion.

As a result, for the purposes of the present invention, the combinations are not exclusively limited to those which are obtained by physical association of the constituents, but also to those which permit a separate administration, which can be simultaneous or spaced out over a period of time.

The compositions according to the invention are preferably compositions which can be administered parentally. However, these compositions may be administered orally, subcutaneously or intraperitoneally in the case of localized regional therapies.

The compositions for parental administration are generally pharmaceutically acceptable, sterile solutions or suspensions which may optionally be prepared as required at the time of use. For the preparation of non-aqueous solutions or suspensions, natural vegetable oils such as olive oil, sesame oil or liquid petroleum or injectable organic esters such as ethyl oleate may be used. The sterile aqueous solutions can consist of a solution of the product in water. The aqueous solutions are suitable for intravenous administration provided the pH is appropriately adjusted and the solution is made isotonic, for example with a sufficient amount of sodium chloride or glucose. The sterilization may be carried out by heating or by any other means which does not adversely affect the composition. The combinations may also take the form of liposomes or the form of an association with carriers as cyclodextrins or polyethylene glycols.

The compositions for oral, subcutaneous or intraperitoneal administration are preferably aqueous suspensions or solutions.

In the combinations according to the invention, the application of the constituents of which may be simultaneous, separate or spaced out over a period of time, it is especially advantageous for the amount of humanized anti-CD38 antibody to represent from 10 to 90 % by weight of the combination, it being possible for this content to vary in accordance with the nature of the associated substance, the efficacy sought and the nature of the cancer to be treated.

The combinations according to the invention are especially useful in the treatment of several types of cancers including (but not limited to) the following: carcinomas and adenocarcinomas, including that of the bladder, breast, colon, head-and-neck, prostate, kidney, liver, lung, ovary, pancreas, stomach, cervix, thyroid and skin, and including squamous cell carcinoma ; hematopoietic tumors of lymphoid lineage, including multiple myeloma, leukemia, acute and chronic lymphocytic (or lymphoid) leukemia, acute and chronic lymphoblastic leukemia, B-cell lymphoma, T-cell lymphoma, non-Hodgkin lymphoma (e.g. Burkitt's lymphoma) ; hematopoietic tumors of myeloid lineage, including acute and chronic myelogenous (myeloid or myelocytic) leukemias, and promyelocytic leukemia; tumors of mesenchymal origin, including fibrosarcoma, osteosarcoma and rhabdomyosarcoma; tumors of the central and peripheral nervous system, including astrocytoma, neuroblastoma, glioma, and schwannomas; and other tumors, including melanoma, teratocarcinoma, xeroderma pigmentosum, keratoacanthoma, and seminoma, and other cancers yet to be determined in which CD38 is expressed. They are mainly useful for treating leukemia, lymphoma and cancers resistant to the commonly used anticancer agents as anti-CD38 antibodies have a unique mechanism of action.

Thus, the invention also encompasses the use of the above combinations for the manufacture of a medicament for the treatment of cancer.

**Example:**

In this example, the effectiveness of an anti-CD38 antibody/cytarabine combination of the invention for tumor growth inhibition was demonstrated in vivo.

The selected tumor model was a transplantable human T-cell acute lymphoblastic leukemia (T-ALL) cell line, DND-41, implanted in SCID mice.

Hu38SB19 was formulated in phosphate buffer saline, without $Ca^{2+}$ and $Mg^{2+}$, pH7.4. Hu38SB19 was administered intravenously on days 18, 21, 24, 27 after tumor implantation.

Palmo Ara-C, a cytarabine derivative suitable for slow-release administration in mice, was formulated in 3 % ethanol, 1 % polysorbate 80, 96 % water, and was administered subcutaneously on days 18, 21, 24, 27 after tumor implantation.

The results of the experiment are reported in Table 1.

Tumor doubling time = 3.4 days.

The following end points have been used:

- Toxicity was declared at dosages inducing ≥ 20 % body weight loss or ≥ 10 % drug death,
- Antitumor efficacy was determined by evaluating log10 cell kill = (T-C) / [3.32 x (tumor doubling time in days)] (T meaning the median time of the treated mice to reach 750 mg and C the median time (26.9 days) of the control mice to reach the same size; tumor-free survivors are excluded from these calculations and are tabulated separately).

No antitumor activity was declared for log cell kill < 0.7, and the treatment was declared highly active for log cell kill $\geq 2.8$

- Tumor Free Survivors (TFS): correspond to complete regression below the limit of palpation (63 mg) for the entire duration of the study (>100 days post last treatment).
- Therapeutic Synergism: a combination has therapeutic synergism if it is more active than the best single agent of the study (by at least 1 log cell kill).

Toxicity for Palmo Ara-C alone was observed at a dose of 96.3 mg/kg/injection, with 6 drug-related deaths out of 6 mice treated. The highest nontoxic dose (HNTD) for Palmo Ara-C was 58.0 mg/kg/inj (total injected dose = 232.0 mg/kg). The 58.0 mg/kg/inj dose was found to be highly active with a $\log_{10}$ cell kill of 5.4 and 3/6 TFS on day 160. The dose below of 36.0 mg/kg/inj (total injected dose = 144.0 mg/kg) was also found to be highly active with a $\log_{10}$ cell kill of 4.3. The lowest dose of 22.3 mg/kg/inj was active with a 2.0 $\log_{10}$ cell kill.

Regarding hu38SB19, the product was well tolerated at a dose of 40 mg/kg/inj. No toxicity was observed, which can be explained by the lack of cross-reactivity of the antibody with murine CD38. The $\log_{10}$ cell kill was 0.5, indicating that hu38DB19 was not active under these conditions.

The combination of Palmo Ara-C at 96.3 mg/kg/inj and hu38SB19 at 40 mg/kg/inj was toxic, with 5 drug-related deaths out of 6 mice treated, i.e. very similar to what was observed with Palmo Ara-C alone at the same dose. The dose of 58.0 mg/kg/inj of Palmo Ara-C with 40 mg/kg/inj of hu38SB19 was considered to be the HNTD. Remarkably, at this dose, 5 out of 6 mice stayed TFS until the end of the study (day 160). The lower dose of 36.0 mg/kg/inj of Palmo Ara-C with 40 mg/kg/inj of hu38SB19 displayed a $\log_{10}$ cell kill of 8.1 and 3/6 TFS in comparison to a 4.3 $\log_{10}$ cell kill and no TFS for the equitoxic dose of Palmo Ara-C alone and was thus considered highly active. An antitumor activity of 2.7 $\log_{10}$ cell kill was recorded for the lowest dose of the combination (in comparison to 2.0 $\log_{10}$ cell kill for the equitoxic dose of Palmo Ara-C alone. We conclude that the combination shows a therapeutic synergism in comparison to the best single agent of the study, Palmo Ara-C.

Table I: Combination of hu38SB19 and Palmo Ara-C against advanced human T-cell acute lymphoblastic leukemia DND-41 implanted in SCID female mice.

| hu38SB19 IV | Palmo Ara-C SC | Schedule in days | Drug death | %BWC at nadir (day) | T-C in days (1000mg) | $\log_{10}$ cell kill gross | TFS tumor free survivor | |
|---|---|---|---|---|---|---|---|---|
| 40.0 (160.0) | - | 18,21,24,27 | 0/6 | -2.1 (19) | 6.1 | 0.5 | 0/6 | HDT - Inactive |
| - | 96.3 (385.2) | 18,21,24,27 | 6/6 | -26.6(31) | - | - | - | Toxic |
| - | 58.0 (232.0) | | 0/6 | -6.1 (31) | 60.5 | 5.4 | 3/6 | HNTD - highly active |
| - | 36.0 (144.0) | | 0/6 | -4.6 (30) | 48.6 | 4.3 | 0/6 | Highly active |
| - | 22.3(89.2) | | 0/6 | -0.5 (32) | 22.1 | 2.0 | 0/6 | Active |

(continued)

| | | Schedule in days | Drug death | %BWC at nadir (day) | T-C in days (1000mg) | $Log_{10}$ cell kill gross | TFS tumor free survivor | |
|---|---|---|---|---|---|---|---|---|
| 40.0 (160.0) | 96.3 (385.2) | 18,21,24,27 | 5/6 | -21.6 (34) | - | - | - | Toxic |
| 40.0 (160.0) | 58.0 (232.0) | | 0/6 | -6.5 (30) | | | 5/6 | HNTD - Highly active |
| 40.0 (160.0) | 36.0 (144.0) | | 0/6 | -3.2 (31 ) | 90.9 | 8.1 | 3/6 | Highly active |
| 40.0 (160.0) | 22.3(89.2) | | 0/6 | -1.7 (19) | 30.8 | 2.7 | 0/6 | active |
| Tumor doubling time = 3.4 days. Median tumor size at start of therapy = 124-136 mg. Time for median control tumor to reach 750 mg = 26.9 days. BWC = body weight change, T-C = tumor growth delay, HDT = highest dose tested, HNTD = highest nontoxic dose, TFS = tumor free survivors, IV = intravenous, SC = subcutaneous,. Formulations: hu38SB19 = phosphate buffer saline without $Ca^{2+}$ and $Mg^{2+}$, pH 7.4, Palmo Ara-C = 3 % ethanol, 1 % polysorbate 80, 96 % water. | | | | | | | | |

**Claims**

1. A pharmaceutical combination comprising an antibody specifically recognizing CD38 and at least cytarabine, wherein said antibody is capable of killing a D38$^+$ cell by apoptosis, antibody-dependent cell-mediated cytotoxicity (ADCC), and complement-dependent cytotoxicity (CDC).

2. The combination of claim 1, wherein said antibody is a humanized antibody.

3. The combination of claim 1 or claim 2, wherein said antibody is a humanized antibody produced by resurfacing.

4. The combination of claim 2 or claim 3 wherein said antibody comprises one or more complementarity-determining region having an amino acid sequence selected from the group consisting of SEQ ID NOS: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 and 36.

5. The combination of claim 2 or claim 3, wherein said antibody is a humanized version of the murine 38SB19 antibody, wherein the heavy chain of said murine 38SB19 antibody comprises three sequential complementarity-determining regions having amino acid sequences represented by SEQ ID NOS: 13, 14, and 15, and wherein the light chain of said 38SB19 antibody comprises three sequential complementarity-determining regions having amino acid sequences represented by SEQ ID NOS: 16, 17, and 18.

6. The combination of claim 5, wherein said 38SB19 antibody is an antibody produced by the hybridoma deposited at the American Type Culture Collectin under accession number PTA-7670.

7. The combination of any one of claims 1 to 4 wherein said antibody comprises at least one heavy chain and at least one light chain, wherein said heavy chain comprises an amino acid sequence represented by SEQ ID NO: 66 and/or said heavy chain comprises three sequential complementarity-determining regions having amino acid sequences represented by SEQ ID NOS: 13, 14, and 15, and wherein said light chain comprises an amino acid sequence selected from the group of SEQ ID NOS: 62 and 64, and/or said light chain comprises three sequential complementarity-determining regions having amino acid sequences represented by SEQ ID NOS: 16, 17, and 18.

8. The combination of claim any one of claims 1 to 4 wherein said antibody comprises at least one heavy chain and at least one light chain, wherein said heavy chain has an amino acid *sequence* represented by SEQ ID NO: 72 and/or said heavy chain comprises three sequential complementarity-determining regions having amino acid sequences represented by SEQ ID NOS: 25, 26, and 27, and wherein said light chain has an amino acid sequence selected from the group of SEQ ID NOS: 68 and 70 and/or said light chain comprises three sequential complementarity-determining regions having amino acid sequences represented by SEQ ID NOS: 28, 29, and 30.

9. A pharmaceutical combination according to any one of claims 1 to 8, wherein said pharmaceutical combination is a pharmaceutical composition comprising said anti-CD38 antibody in combination with cytarabine.

10. The pharmaceutical combination of claim 1 for use in the treatment of cancer.

11. The combination for use of claim 10, wherein said antibody is a humanized antibody.

12. The combination for use of claim 10 or claim 11, wherein said antibody is a humanized antibody produced by resurfacing.

13. The combination for use of claim 11 or claim 12, wherein said antibody is a humanized version of the murine 38SB19 antibody, wherein the heavy chain of said murine 38SB19 antibody comprises three sequential complementarity-determining regions having amino acid sequences represented by SEQ ID NOS: 13, 14, and 15, and wherein the light chain of said 38SB19 antibody comprises three sequential complementarity-determining regions having amino acid sequences represented by SEQ ID NOS: 16, 17, and 18.

14. The combination for use of claim 13, wherein said 38SB19 antibody is an antibody produced by the hybridoma deposited at the American Type Culture Collection under accession number PTA-7670.

15. The combination for use of claim 10 wherein said antibody comprises one or more complementarity-determining region having an amino acid sequence selected from the group consisting of SEQ ID NOS: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 and 36.

16. The combination for use of claim 13 wherein said antibody comprises at least one heavy chain and least one light chain, wherein sajd heavy chain comprises an amino acid sequence represented by SEQ ID NO: 66 and/or said heavy chain comprises three sequential complementarity-determining regions having amino acid sequences represented by SEQ ID NOS: 13, 14, and 15, and wherein said light chain comprises an amino acid sequence selected from the group of SEQ ID NOS: 62 and 64 and/or said light chain comprises three sequential complementarity-determining regions having amino acid sequences represented by SEQ ID NOS: 16, 17, and 18.

17. The combination for use of claim 13 wherein said antibody comprises at least one heavy chain and at least one light chain, wherein said heavy chain comprises an amino acid sequence represented by SEQ ID NO: 72 and/or said heavy chain comprises three sequential complementarity-determining regions having amino acid sequences represented by SEQ ID NOS: 25, 26, and 27, and wherein said light chain comprises an amino acid sequence selected from the group of SEQ ID NOS: 68 and 70 and/or said light chain comprises three sequential complementarity-determining regions having amino acid sequences represented by SEQ ID NOS: 28, 29, and 30.

18. The combination of claim 9 or for use according to claims 10-15, wherein the constituents of said combination are for separate administration.

19. The combination of claim 9 or for use according to claims 10-15, wherein the constituents of said combination are for simultaneous administration.

## Patentansprüche

1. Pharmazeutische Kombination, umfassend einen CD38 spezifisch erkennenden Antikörper und wenigstens Cytarabin, wobei der Antikörper in der Lage ist, eine CD38$^+$-Zelle über Apoptose, antikörperabhängige zellvermittelte Zytotoxizität (ADCC) und komplementabhängige Zytotoxizität (CDC) abzutöten.

2. Kombination nach Anspruch 1, wobei es sich bei dem Antikörper um einen humanisierten Antikörper handelt.

3. Kombination nach Anspruch 1 oder Anspruch 2, wobei es sich bei dem Antikörper um einen mittels Oberflächenveränderung (Resurfacing) hergestellten humanisierten Antikörper handelt.

4. Kombination nach Anspruch 2 oder Anspruch 3, wobei der Antikörper eine oder mehrere Komplementarität bestimmende Regionen mit einer aus der aus SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 und 36 bestehenden Gruppe ausgewählten Aminosäuresequenz umfasst.

5. Kombination nach Anspruch 2 oder Anspruch 3, wobei es sich bei dem Antikörper um eine humanisierte Version des Maus-38SB19-Antikörpers handelt, wobei die schwere Kette des Maus-38SB19-Antikörpers drei aufeinander folgende Komplementarität bestimmende Regionen mit Aminosäuresequenzen gemäß SEQ ID NO: 13, 14 und 15 umfasst und wobei die leichte Kette des Maus-38SB19-Antikörpers drei aufeinander folgende Komplementarität bestimmende Regionen mit Aminosäuresequenzen gemäß SEQ ID NO: 16, 17 und 18 umfasst.

6. Kombination nach Anspruch 5, wobei es sich bei dem 38SB19-Antikörper um einen von dem bei der American Type Culture Collection unter Zugangsnummer PTA-7670 hinterlegten Hybridom produzierten Antikörper handelt.

7. Kombination nach einem der Ansprüche 1 bis 4, wobei der Antikörper wenigstens eine schwere Kette und wenigstens eine leichte Kette umfasst, wobei die schwere Kette eine Aminosäuresequenz gemäß SEQ ID NO: 66 umfasst und/oder die schwere Kette drei aufeinander folgende Komplementarität bestimmende Regionen mit Aminosäuresequenzen gemäß SEQ ID NO: 13, 14 und 15 umfasst und wobei die leichte Kette eine aus der Gruppe SEQ ID NO 62 und 64 ausgewählte Aminosäuresequenz umfasst und/oder die leichte Kette drei aufeinander folgende Komplementarität bestimmende Regionen mit Aminosäuresequenzen gemäß SEQ ID NO: 16, 17 und 18 umfasst.

8. Kombination nach einem der Ansprüche 1 bis 4, wobei der Antikörper wenigstens eine schwere Kette und wenigstens eine leichte Kette umfasst, wobei die schwere Kette eine Aminosäuresequenz gemäß SEQ ID NO: 72 aufweist und/oder die schwere Kette drei aufeinander folgende Komplementarität bestimmende Regionen mit Aminosäuresequenzen gemäß SEQ ID NO: 25, 26 und 27 umfasst und wobei die leichte Kette eine aus der Gruppe SEQ ID NO 68 und 70 ausgewählte Aminosäuresequenz aufweist und/oder die leichte Kette drei aufeinander folgende Komplementarität bestimmende Regionen mit Aminosäuresequenzen gemäß SEQ ID NO: 28, 29 und 30 umfasst.

9. Pharmazeutische Kombination gemäß einem der Ansprüche 1 bis 8, wobei es sich bei der pharmazeutischen Kombination um eine den Anti-CD38-Antikörper in Kombination mit Cytarabin umfassende pharmazeutische Kombination handelt.

10. Pharmazeutische Kombination nach Anspruch 1 zur Verwendung bei der Behandlung von Krebs.

11. Kombination zur Verwendung nach Anspruch 10, wobei es sich bei dem Antikörper um einen humanisierten Antikörper handelt.

12. Kombination zur Verwendung nach Anspruch 10 oder Anspruch 11, wobei es sich bei dem Antikörper um einen mittels Oberflächenveränderung (Resurfacing) hergestellten humanisierten Antikörper handelt.

13. Kombination zur Verwendung nach Anspruch 11 oder Anspruch 12, wobei es sich bei dem Antikörper um eine humanisierte Version des Maus-38SB19-Antikörpers handelt, wobei die schwere Kette des Maus38SB19-Antikörpers drei aufeinander folgende Komplementarität bestimmende Regionen mit Aminosäuresequenzen gemäß SEQ ID NO: 13, 14 und 15 umfasst und wobei die leichte Kette des Maus38SB19-Antikörpers drei aufeinander folgende Komplementarität bestimmende Regionen mit Aminosäuresequenzen gemäß SEQ ID NO: 16, 17 und 18 umfasst.

14. Kombination zur Verwendung nach Anspruch 13, wobei es sich bei dem 38SB19-Antikörper um einen von dem bei der American Type Culture Collection unter Zugangsnummer PTA-7670 hinterlegten Hybridom produzierten Antikörper handelt.

15. Kombination zur Verwendung nach Anspruch 10, wobei der Antikörper eine oder mehrere Komplementarität bestimmende Regionen mit einer aus der aus SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 und 36 bestehenden Gruppe ausgewählten Aminosäuresequenz umfasst.

16. Kombination zur Verwendung nach Anspruch 13, wobei der Antikörper wenigstens eine schwere Kette und wenigstens eine leichte Kette umfasst, wobei die schwere Kette eine Aminosäuresequenz gemäß SEQ ID NO: 66 umfasst und/oder die schwere Kette drei aufeinander folgende Komplementarität bestimmende Regionen mit Aminosäuresequenzen gemäß SEQ ID NO: 13, 14 und 15 umfasst und wobei die leichte Kette eine aus der Gruppe SEQ ID NO 62 und 64 ausgewählte Aminosäuresequenz umfasst und/oder die leichte Kette drei aufeinander folgende Komplementarität bestimmende Regionen mit Aminosäuresequenzen gemäß SEQ ID NO: 16, 17 und 18 umfasst.

17. Kombination zur Verwendung nach Anspruch 13, wobei der Antikörper wenigstens eine schwere Kette und wenigstens eine leichte Kette umfasst, wobei die schwere Kette eine Aminosäuresequenz gemäß SEQ ID NO: 72 umfasst und/oder die schwere Kette drei aufeinander folgende Komplementarität bestimmende Regionen mit Aminosäuresequenzen gemäß SEQ ID NO: 25, 26 und 27 umfasst und wobei die leichte Kette eine aus der Gruppe SEQ ID NO 68 und 70 ausgewählte Aminosäuresequenz umfasst und/oder die leichte Kette drei aufeinander folgende Komplementarität bestimmende Regionen mit Aminosäuresequenzen gemäß SEQ ID NO: 28, 29 und 30 umfasst.

18. Kombination nach Anspruch 9 oder zur Verwendung gemäß Anspruch 10-15, wobei die Bestandteile der Kombination für eine getrennte Verabreichung vorgesehen sind.

19. Kombination nach Anspruch 9 oder zur Verwendung gemäß Anspruch 10-15, wobei die Bestandteile der Kombination für eine gleichzeitige Verabreichung vorgesehen sind.

**Revendications**

1. Combinaison pharmaceutique comprenant un anticorps reconnaissant spécifiquement CD38 et au moins la cytarabine, où ledit anticorps peut détruire une cellule CD38[+] par apoptose, cytotoxicité faisant intervenir les cellules dépendant des anticorps (ADCC) et phytotoxicité dépendant du complément(CDC).

**2.** Combinaison selon la revendication 1, où ledit anticorps est un anticorps humanisé.

**3.** Combinaison selon la revendication 1 ou la revendication 2, où ledit anticorps est un anticorps humanisé produit par resurfaçage.

**4.** Combinaison selon la revendication 2 ou la revendication 3, où ledit anticorps comprend une ou plusieurs régions déterminant la complémentarité présentant une séquence d'acides aminés choisie dans le groupe constitué par les SEQ ID NOS: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 et 36.

**5.** Combinaison selon la revendication 2 ou la revendication 3, où ledit anticorps est une version humanisée de l'anticorps murin 38SB19, où la chaîne lourde dudit anticorps murin 38SB19 comprend trois régions séquentielles déterminant la complémentarité dont les séquences d'acides aminés sont représentées par les SEQ ID NOS : 13, 14 et 15, et où la chaîne légère dudit anticorps 38SB19 comprend trois régions séquentielles déterminant la complémentarité dont les séquences d'acides aminés sont représentées par les SEQ ID NOS : 16, 17 et 18.

**6.** Combinaison selon la revendication 5, où ledit anticorps 38SB19 est un anticorps produit par l'hybridome déposé auprès de l'American Type Culture Collection sous le numéro d'accès PTA-7670.

**7.** Combinaison selon l'une quelconque des revendications 1 à 4, où ledit anticorps comprend au moins une chaîne lourde et au moins une chaîne légère, où ladite chaîne lourde comprend une séquence d'acides aminés représentés par SEQ ID NO : 66 et/ou ladite chaîne lourde comprend trois régions séquentielles déterminant la complémentarité dont les séquences d'acides aminés sont représentées par les SEQ ID NOS : 13, 14 et 15, et où ladite chaîne légère comprend une séquence d'acides aminés choisie dans le groupe constitué par les SEQ ID NOS : 62 et 64, et/ou ladite chaîne légère comprend trois régions séquentielles déterminant la complémentarité dont les séquences d'acides aminés sont représentées par les SEQ ID NOS : 16, 17 et 18.

**8.** Combinaison selon l'une quelconque des revendications 1 à 4, où ledit anticorps comprend au moins une chaîne lourde et au moins une chaîne légère, où ladite chaîne lourde comprend une séquence d'acides aminés représentés par SEQ ID NO : 72 et/ou ladite chaîne lourde comprend trois régions séquentielles déterminant la complémentarité dont les séquences d'acides aminés sont représentées par les SEQ ID NOS : 25, 26 et 27, et où ladite chaîne légère comprend une séquence d'acides aminés choisie dans le groupe constitué par les SEQ ID NOS : 68 et 70, et/ou ladite chaîne légère comprend trois régions séquentielles déterminant la complémentarité dont les séquences d'acides aminés sont représentées par les SEQ ID NOS : 28, 29 et 30.

**9.** Combinaison pharmaceutique selon l'une quelconque des revendications 1 à 8, où ladite combinaison pharmaceutique est une composition pharmaceutique comprenant ledit anticorps anti-CD38 en combinaison avec la cytarabine.

**10.** Combinaison pharmaceutique selon la revendication 1, destinée à une utilisation dans le traitement du cancer.

**11.** Combinaisons selon la revendication 10, où ledit anticorps est un anticorps humanisé.

**12.** Combinaison destinée à être utilisée selon la revendication 10 ou la revendication 11, où ledit anticorps est un anticorps humanisé produit par resurfaçage.

**13.** Combinaison destinée à être utilisée selon la revendication 11 ou la revendication 12, où ledit anticorps est une version humanisée de l'anticorps murin 38SB19, où la chaîne lourde dudit anticorps murin 38SB19 comprend trois régions séquentielles déterminant la complémentarité dont les séquences d'acides aminés sont représentées par les SEQ ID NOS: 13, 14 et 15, et où la chaîne légère dudit anticorps 38SB19 comprend trois régions séquentielles déterminant la complémentarité dont les séquences d'acides aminés sont représentées par les SEQ ID NOS : 16, 17 et 18.

**14.** Combinaison destinée à être utilisée selon la revendication 13, où ledit anticorps 38SB19 est un anticorps produit par l'hybridome déposé auprès de l'American Type Culture Collection sous le numéro d'accès PTA-7670.

**15.** Combinaison destinée à être utilisée selon la revendication 10, où ledit anticorps comprend une ou plusieurs régions déterminant la complémentarité présentant une séquence d'acides aminés choisie dans le groupe constitué par les SEQ ID NOS: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 et 36.

16. Combinaison destinée à être utilisée selon la revendication 13, où ledit anticorps comprend au moins une chaîne lourde et au moins une chaîne légère, où ladite chaîne lourde comprend une séquence d'acides aminés représentée par SEQ ID NO : 66 et/ou ladite chaîne lourde comprend trois régions séquentielles déterminant la complémentarité dont les séquences d'acides aminés sont représentées par les SEQ ID NOS : 13, 14 et 15, et où ladite chaîne légère comprend une séquence d'acides aminés choisie dans le groupe constitué par les SEQ ID NOS : 62 et 64, et/ou ladite chaîne légère comprend trois régions séquentielles déterminant la complémentarité dont les séquences d'acides aminés sont représentées par les SEQ ID NOS : 16, 17 et 18.

17. Combinaison destinée à être utilisée selon la revendication 13, où ledit anticorps comprend au moins une chaîne lourde et au moins une chaîne légère, où ladite chaîne lourde comprend une séquence d'acides aminés représentée par la SEQ ID NO : 72 et/ou ladite chaîne lourde comprend trois régions séquentielles déterminant la complémentarité dont les séquences d'acides aminés sont représentées par les SEQ ID NOS : 25, 26 et 27, et où ladite chaîne légère comprend une séquence d'acides aminés choisie dans le groupe constitué par les SEQ ID NOS : 68 et 70, et/ou ladite chaîne légère comprend trois régions séquentielles déterminant la complémentarité dont les séquences d'acides aminés sont représentées par les SEQ ID NOS : 28, 29 et 30.

18. Combinaison selon la revendication 9 ou destinée à être utilisée selon les revendications 10 à 15, où les constituants de ladite combinaison sont destinés à une administration séparée.

19. Combinaison selon la revendication 9 ou pour utilisation selon les revendications 10 à 15, où les constituants de ladite combinaison sont destinés à une administration simultanée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008047242 A
- WO 9222653 A
- US 5639641 A
- EP 0239400 A
- WO 9109967 A
- US 5530101 A
- US 5585089 A
- EP 0592106 A
- EP 0519596 A
- US 5565332 A
- US 2008074381 W
- US 4444887 A
- US 4716111 A
- US 5545806 A
- US 5814318 A
- WO 9846645 A
- WO 9850433 A
- WO 9824893 A
- WO 9816654 A
- WO 9634096 A
- WO 9633735 A
- WO 9110741 A
- FR 2008113

### Non-patent literature cited in the description

- **PADLAN E. A.** *Molecular Immunology,* 1991, vol. 28 (4/5), 489-498
- **STUDNICKA G. M. et al.** *Protein Engineering,* 1994, vol. 7 (6), 805-814
- **ROGUSKA M.A. et al.** *PNAS,* 1994, vol. 91, 969-973
- **T.H. CORBETT et al.** *Cancer,* 1977, vol. 40, 2660-2680
- Cancer Drug Development. **F.M. SCHABEL et al.** Methods in Cancer Research. Academic Press Inc, 1979, vol. 17, 3-51

SEQUENCE LISTING

**[0047]**

<110> SANOFI-AVENTIS

<120> ANTITUMOR COMBINATIONS CONTAINING ANTIBODIES RECOGNIZING SPECIFICALLY CD38 AND CYTARABINE

<130> FR2008-113

<160> 80

<170> PatentIn version 3.3

<210> 1
<211> 5
<212> PRT
<213> Mus sp.

<400> 1

```
Ser Tyr Gly Met Asn
1               5
```

<210> 2
<211> 17
<212> PRT
<213> Mus sp.

<400> 2

Trp Ile Asn Thr Tyr Thr Gly Glu Pro Thr Tyr Ala Asp Asp Phe Lys
1               5               10              15

Gly

<210> 3
<211> 5
<212> PRT
<213> Mus sp.

<400> 3

Arg Gly Phe Ala Tyr
1               5

<210> 4
<211> 15
<212> PRT
<213> Mus sp.

<400> 4

Arg Ala Ser Glu Ser Val Glu Ile Tyr Gly Asn Gly Phe Met Asn
1               5               10              15

<210> 5
<211> 7
<212> PRT
<213> Mus sp.

<400> 5

Arg Ala Ser Asn Leu Glu Ser
1               5

<210> 6
<211> 9
<212> PRT
<213> Mus sp.

<400> 6

Gln Gln Ile Asn Glu Asp Pro Phe Thr
1               5

<210> 7
<211> 5
<212> PRT
<213> Mus sp.

<400> 7

```
Asn Ser Gly Met Asn
1               5
```

<210> 8
<211> 17
<212> PRT
<213> Mus sp.

<400> 8

```
Trp Ile Asn Thr Tyr Thr Gly Glu Pro Thr Tyr Ala Asp Asp Phe Lys
1               5                   10                  15

Gly
```

<210> 9
<211> 5
<212> PRT
<213> Mus sp.

<400> 9

```
Arg Gly Phe Val Tyr
1               5
```

<210> 10
<211> 15
<212> PRT
<213> Mus sp.

<400> 10

```
Arg Ala Ser Glu Ser Val Ala Ile Tyr Gly Asn Ser Phe Leu Lys

    1           5                   10                  15
```

<210> 11
<211> 7
<212> PRT
<213> Mus sp.

<400> 11

```
Arg Ala Ser Asn Leu Glu Ser
1               5
```

<210> 12
<211> 9
<212> PRT
<213> Mus sp.

<400> 12

```
                    Gln Gln Ile Asn Glu Asp Pro Tyr Thr
                    1               5
```

<210> 13
<211> 5
<212> PRT
<213> Mus sp.

<400> 13

```
                        Asp Tyr Trp Met Gln
                        1               5
```

<210> 14
<211> 17
<212> PRT
<213> Mus sp.

<400> 14

```
        Thr Ile Tyr Pro Gly Asp Gly Asp Thr Gly Tyr Ala Gln Lys Phe Lys
        1               5                   10                  15
```

```
        Gly
```

<210> 15
<211> 11
<212> PRT
<213> Mus sp.

<400> 15

```
                    Gly Asp Tyr Tyr Gly Ser Asn Ser Leu Asp Tyr
                    1               5                   10
```

<210> 16
<211> 11
<212> PRT
<213> Mus sp.

<400> 16

```
                    Lys Ala Ser Gln Asp Val Ser Thr Val Val Ala
                    1               5                   10
```

<210> 17
<211> 7
<212> PRT
<213> Mus sp.

<400> 17

```
                              Ser Ala Ser Tyr Arg Tyr Ile
                              1               5
```

<210> 18
<211> 9
<212> PRT
<213> Mus sp.

<400> 18

```
                    Gln Gln His Tyr Ser Pro Pro Tyr Thr
                    1               5
```

<210> 19
<211> 5
<212> PRT
<213> Mus sp.

<400> 19

```
                              Gly Ser Trp Met Asn
                              1               5
```

<210> 20
<211> 17
<212> PRT
<213> Mus sp.

<400> 20

```
        Arg Ile Tyr Pro Gly Asp Gly Asp Ile Ile Tyr Asn Gly Asn Phe Arg
        1               5                   10                  15

        Asp
```

<210> 21
<211> 10
<212> PRT
<213> Mus sp.

<400> 21

```
                    Trp Gly Thr Phe Thr Pro Ser Phe Asp Tyr


                    1               5                       10
```

<210> 22
<211> 11
<212> PRT

<213> Mus sp.

<400> 22

```
Lys Ala Ser Gln Asp Val Val Thr Ala Val Ala
1               5                   10
```

<210> 23
<211> 7
<212> PRT
<213> Mus sp.

<400> 23

```
Ser Ala Ser His Arg Tyr Thr
1               5
```

<210> 24
<211> 9
<212> PRT
<213> Mus sp.

<400> 24

```
Gln Gln His Tyr Thr Thr Pro Thr Thr
1               5
```

<210> 25
<211> 5
<212> PRT
<213> Mus sp.

<400> 25

```
Ser Tyr Thr Leu Ser
1               5
```

<210> 26
<211> 17
<212> PRT
<213> Mus sp.

<400> 26

```
Thr Ile Ser Ile Gly Gly Arg Tyr Thr Tyr Tyr Pro Asp Ser Val Glu
1               5                   10                  15

Gly
```

<210> 27
<211> 8
<212> PRT

<213> Mus sp.

<400> 27

Asp Phe Asn Gly Tyr Ser Asp Phe
1               5

<210> 28
<211> 11
<212> PRT
<213> Mus sp.

<400> 28

Lys Ala Ser Gln Val Val Gly Ser Ala Val Ala
1               5                   10

<210> 29
<211> 7
<212> PRT
<213> Mus sp.

<400> 29

Trp Ala Ser Thr Arg His Thr
1               5

<210> 30
<211> 9
<212> PRT
<213> Mus sp.

<400> 30

Gln Gln Tyr Asn Ser Tyr Pro Tyr Thr
1               5

<210> 31
<211> 5
<212> PRT
<213> Mus sp.

<400> 31

Asn Phe Gly Met His
1               5

<210> 32
<211> 17
<212> PRT
<213> Mus sp.

<400> 32

```
Tyr Ile Arg Ser Gly Ser Gly Thr Ile Tyr Tyr Ser Asp Thr Val Lys
1               5                   10                  15

Gly
```

<210> 33
<211> 11
<212> PRT
<213> Mus sp.

<400> 33

```
Ser Tyr Tyr Asp Phe Gly Ala Trp Phe Ala Tyr
1               5                   10
```

<210> 34
<211> 11
<212> PRT
<213> Mus sp.

<400> 34

```
Lys Ala Ser Gln Asn Val Gly Thr Asn Val Ala
1               5                   10
```

<210> 35
<211> 7
<212> PRT
<213> Mus sp.

<400> 35

```
Ser Ala Ser Ser Arg Tyr Ser
1               5
```

<210> 36
<211> 9
<212> PRT
<213> Mus sp.

<400> 36

```
Gln Gln Tyr Asn Ser Tyr Pro Leu Thr
1               5
```

<210> 37
<211> 336
<212> DNA
<213> Mus sp.

<220>
<221> CDS

<222> (1)..(336)

<400> 37

```
aac att gtg ctg acc caa tct cca gct tct ttg gct gtg tct ctt ggg      48
Asn Ile Val Leu Thr Gln Ser Pro Ala Ser Leu Ala Val Ser Leu Gly
1                   5                   10                  15

cag agg gcc acc ata tcc tgc aga gcc agt gaa agt gtt gag att tat      96
Gln Arg Ala Thr Ile Ser Cys Arg Ala Ser Glu Ser Val Glu Ile Tyr
                20                  25                  30

ggc aat ggt ttt atg aac tgg ttc cag cag aaa cca gga cag cca ccc     144
Gly Asn Gly Phe Met Asn Trp Phe Gln Gln Lys Pro Gly Gln Pro Pro
            35                  40                  45

aaa ctc ctc atc tat cgt gca tcc aac cta gaa tct ggg atc cct gcc     192
Lys Leu Leu Ile Tyr Arg Ala Ser Asn Leu Glu Ser Gly Ile Pro Ala
        50                  55                  60

agg ttc agt ggc agt ggg tct agg aca gag ttc acc ctc acc att gat     240
Arg Phe Ser Gly Ser Gly Ser Arg Thr Glu Phe Thr Leu Thr Ile Asp
65                  70                  75                  80

cct gtg gag gct gat gat gtt gca acc tat tac tgt caa caa att aat     288
Pro Val Glu Ala Asp Asp Val Ala Thr Tyr Tyr Cys Gln Gln Ile Asn
                85                  90                  95

gag gat cca ttc acg ttc ggc tcg ggg aca aag ttg gaa ata aaa cgg     336
Glu Asp Pro Phe Thr Phe Gly Ser Gly Thr Lys Leu Glu Ile Lys Arg
            100                 105                 110
```

<210> 38
<211> 112
<212> PRT
<213> Mus sp.

<400> 38

EP 2 370 096 B1

```
Asn Ile Val Leu Thr Gln Ser Pro Ala Ser Leu Ala Val Ser Leu Gly
1               5               10              15

Gln Arg Ala Thr Ile Ser Cys Arg Ala Ser Glu Ser Val Glu Ile Tyr
            20              25              30

Gly Asn Gly Phe Met Asn Trp Phe Gln Gln Lys Pro Gly Gln Pro Pro
            35              40              45

Lys Leu Leu Ile Tyr Arg Ala Ser Asn Leu Glu Ser Gly Ile Pro Ala
        50              55              60

Arg Phe Ser Gly Ser Gly Ser Arg Thr Glu Phe Thr Leu Thr Ile Asp
65              70              75              80

Pro Val Glu Ala Asp Asp Val Ala Thr Tyr Tyr Cys Gln Gln Ile Asn
                85              90              95

Glu Asp Pro Phe Thr Phe Gly Ser Gly Thr Lys Leu Glu Ile Lys Arg
            100             105             110
```

<210> 39
<211> 336
<212> DNA
<213> Mus sp.

<220>
<221> CDS
<222> (1)..(336)

<400> 39

```
gac att gta ctg acc caa tct cca gct tct ttg gct gtg tct cta ggg          48
Asp Ile Val Leu Thr Gln Ser Pro Ala Ser Leu Ala Val Ser Leu Gly
1               5                   10                  15

cag agg gcc acc ata tcc tgc aga gcc agt gag agt gtt gct att tat          96
Gln Arg Ala Thr Ile Ser Cys Arg Ala Ser Glu Ser Val Ala Ile Tyr
            20                  25                  30

ggc aat agt ttt ctg aaa tgg ttc cag cag aaa ccg gga cag cca ccc         144
Gly Asn Ser Phe Leu Lys Trp Phe Gln Gln Lys Pro Gly Gln Pro Pro
                35                  40                  45

aaa ctc ctc atc tat cgt gca tcc aac cta gaa tct ggg atc cct gcc         192
Lys Leu Leu Ile Tyr Arg Ala Ser Asn Leu Glu Ser Gly Ile Pro Ala
        50                  55                  60

agg ttc agt ggc agt ggg tct ggg aca gac ttc acc ctc acc att aat         240
Arg Phe Ser Gly Ser Gly Ser Gly Thr Asp Phe Thr Leu Thr Ile Asn
65                  70                  75                  80

cct gtg gag gct gat gat gtt gca acc tat tac tgt cag caa att aat         288
Pro Val Glu Ala Asp Asp Val Ala Thr Tyr Tyr Cys Gln Gln Ile Asn
                85                  90                  95

gag gat ccg tac acg ttc gga ggg ggg acc aag ctg gaa ata aaa cgg        336
Glu Asp Pro Tyr Thr Phe Gly Gly Gly Thr Lys Leu Glu Ile Lys Arg
                100                 105                 110
```

<210> 40
<211> 112
<212> PRT
<213> Mus sp.

<400> 40

```
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ala Ile Tyr
            20                  25                  30

Gly Asn Ser Phe Leu Lys Trp Phe Gln Gln Lys Pro Gly Gln Pro Pro
                35                  40                  45

Lys Leu Leu Ile Tyr Arg Ala Ser Asn Leu Glu Ser Gly Ile Pro Ala
    50                  55                  60

Arg Phe Ser Gly Ser Gly Ser Gly Thr Asp Phe Thr Leu Thr Ile Asn
65                  70                  75                  80

Pro Val Glu Ala Asp Asp Val Ala Thr Tyr Tyr Cys Gln Gln Ile Asn
                85                  90                  95

Glu Asp Pro Tyr Thr Phe Gly Gly Gly Thr Lys Leu Glu Ile Lys Arg


        100                 105                 110
```

<210> 41
<211> 324
<212> DNA
<213> Mus sp.

<220>
<221> CDS
<222> (1)..(324)

<400> 41

```
gac att gtg atg gcc cag tct cac aaa ttc atg tcc aca tca gtt gga        48
Asp Ile Val Met Ala Gln Ser His Lys Phe Met Ser Thr Ser Val Gly
1               5                   10                  15

gac agg gtc agc atc acc tgc aag gcc agt cag gat gtg agt act gtt        96
Asp Arg Val Ser Ile Thr Cys Lys Ala Ser Gln Asp Val Ser Thr Val
            20                  25                  30

gtg gcc tgg tat caa cag aaa cca gga caa tct cct aaa cga ctg att       144
Val Ala Trp Tyr Gln Gln Lys Pro Gly Gln Ser Pro Lys Arg Leu Ile
        35                  40                  45

tac tcg gca tcc tat cgg tat att gga gtc cct gat cgc ttc act ggc       192
Tyr Ser Ala Ser Tyr Arg Tyr Ile Gly Val Pro Asp Arg Phe Thr Gly
        50                  55                  60

agt gga tct ggg acg gat ttc act ttc acc atc agc agt gtg cag gct       240
Ser Gly Ser Gly Thr Asp Phe Thr Phe Thr Ile Ser Ser Val Gln Ala
65              70                  75                  80

gaa gac ctg gca gtt tat tac tgt cag caa cat tat agt cct ccg tac       288
Glu Asp Leu Ala Val Tyr Tyr Cys Gln Gln His Tyr Ser Pro Pro Tyr
                85                  90                  95

acg ttc gga ggg ggg acc aag ctg gaa ata aaa cgg                       324
Thr Phe Gly Gly Gly Thr Lys Leu Glu Ile Lys Arg
            100                 105
```

<210> 42
<211> 108
<212> PRT
<213> Mus sp.

<400> 42

```
    Asp Ile Val Met Ala Gln Ser His Lys Phe Met Ser Thr Ser Val Gly
    1               5                   10                  15


    Asp Arg Val Ser Ile Thr Cys Lys Ala Ser Gln Asp Val Ser Thr Val
                20                  25                  30


    Val Ala Trp Tyr Gln Gln Lys Pro Gly Gln Ser Pro Lys Arg Leu Ile
            35                  40                  45


    Tyr Ser Ala Ser Tyr Arg Tyr Ile Gly Val Pro Asp Arg Phe Thr Gly
            50                  55                  60
```

EP 2 370 096 B1

```
        Ser Gly Ser Gly Thr Asp Phe Thr Phe Thr Ile Ser Ser Val Gln Ala
        65              70                  75                  80


        Glu Asp Leu Ala Val Tyr Tyr Cys Gln Gln His Tyr Ser Pro Pro Tyr
                        85                  90                  95


        Thr Phe Gly Gly Gly Thr Lys Leu Glu Ile Lys Arg
                    100                 105
```

<210> 43
<211> 324
<212> DNA
<213> Mus sp.

<220>
<221> CDS
<222> (1)..(324)

<400> 43

```
    gac att gtg atg acc cag tct cac aaa ttc ttg tcc aca tca gtt gga        48
    Asp Ile Val Met Thr Gln Ser His Lys Phe Leu Ser Thr Ser Val Gly
    1               5                   10                  15

    gac agg gtc agt atc acc tgc aag gcc agt cag gat gtg gtt act gct        96
    Asp Arg Val Ser Ile Thr Cys Lys Ala Ser Gln Asp Val Val Thr Ala
                20                  25                  30

    gtt gcc tgg ttt caa cag aaa cca gga caa tct cca aaa cta ctg att       144
    Val Ala Trp Phe Gln Gln Lys Pro Gly Gln Ser Pro Lys Leu Leu Ile
            35                  40                  45

    tat tcg gca tcc cac cgg tac act gga gtc cct gat cgc ttc act ggc       192
    Tyr Ser Ala Ser His Arg Tyr Thr Gly Val Pro Asp Arg Phe Thr Gly
        50                  55                  60

    agt gga tct ggg aca gat ttc act ttc acc atc atc agt gtg cag gct       240
    Ser Gly Ser Gly Thr Asp Phe Thr Phe Thr Ile Ile Ser Val Gln Ala
    65              70                  75                  80

    gaa gac ctg gca gtt tat tac tgt caa caa cat tat act act ccc acg       288
    Glu Asp Leu Ala Val Tyr Tyr Cys Gln Gln His Tyr Thr Thr Pro Thr
                    85                  90                  95

    acg ttc ggt gga ggc acc aag ctg gac ttc aga cgg                       324
    Thr Phe Gly Gly Gly Thr Lys Leu Asp Phe Arg Arg
                    100                 105
```

<210> 44
<211> 108
<212> PRT
<213> Mus sp.

<400> 44

20

```
Asp Ile Val Met Thr Gln Ser His Lys Phe Leu Ser Thr Ser Val Gly
1               5                   10                  15

Asp Arg Val Ser Ile Thr Cys Lys Ala Ser Gln Asp Val Val Thr Ala
            20                  25                  30

Val Ala Trp Phe Gln Gln Lys Pro Gly Gln Ser Pro Lys Leu Leu Ile
        35                  40                  45

Tyr Ser Ala Ser His Arg Tyr Thr Gly Val Pro Asp Arg Phe Thr Gly
    50                  55                  60

Ser Gly Ser Gly Thr Asp Phe Thr Phe Thr Ile Ile Ser Val Gln Ala
65                  70                  75                  80

Glu Asp Leu Ala Val Tyr Tyr Cys Gln Gln His Tyr Thr Thr Pro Thr
                85                  90                  95

Thr Phe Gly Gly Gly Thr Lys Leu Asp Phe Arg Arg
            100                 105
```

<210> 45
<211> 324
<212> DNA
<213> Mus sp.

<220>
<221> CDS
<222> (1)..(324)

<400> 45

```
gac act gtg atg acc cag tct cac aaa ttc ata tcc aca tca gtt gga        48
Asp Thr Val Met Thr Gln Ser His Lys Phe Ile Ser Thr Ser Val Gly
1               5                   10                  15

gac agg gtc agc atc acc tgc aag gcc agt cag gtt gtg ggt agt gct        96
Asp Arg Val Ser Ile Thr Cys Lys Ala Ser Gln Val Val Gly Ser Ala
            20                  25                  30

gta gcc tgg tat caa cag aaa cca ggg caa tct cct aaa cta ctg att       144
Val Ala Trp Tyr Gln Gln Lys Pro Gly Gln Ser Pro Lys Leu Leu Ile
        35                  40                  45

tac tgg gca tcc acc cgg cac act gga gtc cct gat cgc ttc aca ggc       192
Tyr Trp Ala Ser Thr Arg His Thr Gly Val Pro Asp Arg Phe Thr Gly
        50                  55                  60

agt gga tct ggg aca gat ttc act ctc acc att agc aat gtg cag tct       240
Ser Gly Ser Gly Thr Asp Phe Thr Leu Thr Ile Ser Asn Val Gln Ser
65                  70                  75                  80

gaa gac ttg gca gat tat ttc tgt cag caa tat aac agc tat ccg tac       288
Glu Asp Leu Ala Asp Tyr Phe Cys Gln Gln Tyr Asn Ser Tyr Pro Tyr
                85                  90                  95

acg ttc gga ggg ggg acc aag ctg gaa ata aaa cgg                       324
Thr Phe Gly Gly Gly Thr Lys Leu Glu Ile Lys Arg
            100                 105
```

<210> 46
<211> 108
<212> PRT
<213> Mus sp.

<400> 46

```
Asp Thr Val Met Thr Gln Ser His Lys Phe Ile Ser Thr Ser Val Gly
1               5                   10                  15


Asp Arg Val Ser Ile Thr Cys Lys Ala Ser Gln Val Val Gly Ser Ala
            20                  25                  30


Val Ala Trp Tyr Gln Gln Lys Pro Gly Gln Ser Pro Lys Leu Leu Ile
            35                  40                  45


Tyr Trp Ala Ser Thr Arg His Thr Gly Val Pro Asp Arg Phe Thr Gly
        50                  55                  60


Ser Gly Ser Gly Thr Asp Phe Thr Leu Thr Ile Ser Asn Val Gln Ser
65                  70                  75                  80


Glu Asp Leu Ala Asp Tyr Phe Cys Gln Gln Tyr Asn Ser Tyr Pro Tyr
                85                  90                  95


Thr Phe Gly Gly Gly Thr Lys Leu Glu Ile Lys Arg
            100                 105
```

<210> 47
<211> 324
<212> DNA
<213> Mus sp.

<220>
<221> CDS
<222> (1)..(324)

<400> 47

```
gac att gtg atg acc cag tct caa aaa ttc atg tcc aca tca gta gga         48
Asp Ile Val Met Thr Gln Ser Gln Lys Phe Met Ser Thr Ser Val Gly
1               5                   10                  15

gac agg gtc agc gtc acc tgc aag gcc agt cag aat gtg ggt act aat         96
Asp Arg Val Ser Val Thr Cys Lys Ala Ser Gln Asn Val Gly Thr Asn
            20                  25                  30

gtt gcc tgg tat caa cac aaa cca gga caa tcc cct aaa ata atg att        144
Val Ala Trp Tyr Gln His Lys Pro Gly Gln Ser Pro Lys Ile Met Ile
        35                  40                  45

tat tcg gcg tcc tcc cgg tac agt gga gtc cct gat cgc ttc aca ggc        192
Tyr Ser Ala Ser Ser Arg Tyr Ser Gly Val Pro Asp Arg Phe Thr Gly
    50                  55                  60

agt gga tct ggg aca ctt ttc act ctc acc atc aac aat gtg cag tct        240
Ser Gly Ser Gly Thr Leu Phe Thr Leu Thr Ile Asn Asn Val Gln Ser
65                  70                  75                  80

gaa gac ttg gca gag tat ttc tgt cag caa tat aac agc tat cct ctc        288
Glu Asp Leu Ala Glu Tyr Phe Cys Gln Gln Tyr Asn Ser Tyr Pro Leu
                85                  90                  95

acg ttc ggc tcg ggg aca aag ttg gaa ata aaa cgg                        324
Thr Phe Gly Ser Gly Thr Lys Leu Glu Ile Lys Arg
            100                 105
```

<210> 48
<211> 108
<212> PRT
<213> Mus sp.

<400> 48

```
Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Asn
                20                  25                  30

Val Ala Trp Tyr Gln His Lys Pro Gly Gln Ser Pro Lys Ile Met Ile
            35                  40                  45

Tyr Ser Ala Ser Ser Arg Tyr Ser Gly Val Pro Asp Arg Phe Thr Gly
        50                  55                  60

Ser Gly Ser Gly Thr Leu Phe Thr Leu Thr Ile Asn Asn Val Gln Ser
65                  70                  75                  80

Glu Asp Leu Ala Glu Tyr Phe Cys Gln Gln Tyr Asn Ser Tyr Pro Leu
                85                  90                  95

Thr Phe Gly Ser Gly Thr Lys Leu Glu Ile Lys Arg
            100                 105
```

<210> 49
<211> 342
<212> DNA
<213> Mus sp.

<220>
<221> CDS
<222> (1)..(342)

<400> 49

```
cag atc cag ttg gtg cag tct gga cct gag ctg aag aag cct gga gag        48
Gln Ile Gln Leu Val Gln Ser Gly Pro Glu Leu Lys Lys Pro Gly Glu
1               5                   10                  15

aca gtc aag atc tcc tgc aag gct tct ggg tat acc ctc aca agc tac        96
Thr Val Lys Ile Ser Cys Lys Ala Ser Gly Tyr Thr Leu Thr Ser Tyr
                20                  25                  30

gga atg aac tgg gtg aag cag gct cca gga aag ggt tta aag tgg atg       144
```

```
    Gly Met Asn Trp Val Lys Gln Ala Pro Gly Lys Gly Leu Lys Trp Met
            35                  40                  45

    ggc tgg ata aac acc tac act gga gaa cca aca tat gct gat gac ttt        192
    Gly Trp Ile Asn Thr Tyr Thr Gly Glu Pro Thr Tyr Ala Asp Asp Phe
            50                  55                  60

    aag gga cgt ttt gcc ttc tct ttg gaa acc tct gcc agc act gcc ttt        240
    Lys Gly Arg Phe Ala Phe Ser Leu Glu Thr Ser Ala Ser Thr Ala Phe
    65                  70                  75                  80

    ttg cag atc aac aac ctc aaa aat gag gac acg gct aca tat ttc tgt        288
    Leu Gln Ile Asn Asn Leu Lys Asn Glu Asp Thr Ala Thr Tyr Phe Cys
                        85                  90                  95

    gta aga cgc ggg ttt gct tac tgg ggc caa ggg act ctg gtc act gtc        336
    Val Arg Arg Gly Phe Ala Tyr Trp Gly Gln Gly Thr Leu Val Thr Val
                    100                 105                 110

    tct gca                                                                 342
    Ser Ala
```

<210> 50
<211> 114
<212> PRT
<213> Mus sp.

<400> 50

```
Gln Ile Gln Leu Val Gln Ser Gly Pro Glu Leu Lys Lys Pro Gly Glu
1               5               10                  15

Thr Val Lys Ile Ser Cys Lys Ala Ser Gly Tyr Thr Leu Thr Ser Tyr
            20              25                  30

Gly Met Asn Trp Val Lys Gln Ala Pro Gly Lys Gly Leu Lys Trp Met
            35              40                  45

Gly Trp Ile Asn Thr Tyr Thr Gly Glu Pro Thr Tyr Ala Asp Asp Phe
        50              55                  60

Lys Gly Arg Phe Ala Phe Ser Leu Glu Thr Ser Ala Ser Thr Ala Phe
65              70                  75                      80

Leu Gln Ile Asn Asn Leu Lys Asn Glu Asp Thr Ala Thr Tyr Phe Cys
                85                  90                      95

Val Arg Arg Gly Phe Ala Tyr Trp Gly Gln Gly Thr Leu Val Thr Val
            100                 105                 110

Ser Ala
```

<210> 51
<211> 342
<212> DNA
<213> Mus sp.

<220>
<221> CDS
<222> (1)..(342)

<400> 51

```
cag atc cag ttg gtg cag tct gga cct gag ctg aag aag cct gga gag        48
Gln Ile Gln Leu Val Gln Ser Gly Pro Glu Leu Lys Lys Pro Gly Glu
1               5                   10                  15

aca gtc aag atc tcc tgc aag gct tct ggg tat acc ttc aca aac tct        96
Thr Val Lys Ile Ser Cys Lys Ala Ser Gly Tyr Thr Phe Thr Asn Ser
                20                  25                  30

gga atg aac tgg gtg aag cag gct cca gga aag ggt tta aag tgg atg       144
Gly Met Asn Trp Val Lys Gln Ala Pro Gly Lys Gly Leu Lys Trp Met
            35                  40                  45

ggc tgg ata aac acc tac act gga gag ccg aca tat gct gat gac ttc       192
Gly Trp Ile Asn Thr Tyr Thr Gly Glu Pro Thr Tyr Ala Asp Asp Phe
        50                  55                  60

aag gga cgg ttt gcc ttc tct ttg gaa acc tct gcc agc tct gcc tat       240
Lys Gly Arg Phe Ala Phe Ser Leu Glu Thr Ser Ala Ser Ser Ala Tyr
65                  70                  75                  80

ttg cag atc agt aac ctc aaa aat gag gac acg gct aca tat ttc tgt       288
Leu Gln Ile Ser Asn Leu Lys Asn Glu Asp Thr Ala Thr Tyr Phe Cys
                85                  90                  95

gca aga agg ggt ttt gtt tac tgg ggc caa ggg act ctg gta act gtc       336
Ala Arg Arg Gly Phe Val Tyr Trp Gly Gln Gly Thr Leu Val Thr Val
                100                 105                 110

tct gca                                                               342
Ser Ala
```

<210> 52
<211> 114
<212> PRT
<213> Mus sp.

<400> 52

```
        Gln Ile Gln Leu Val Gln Ser Gly Pro Glu Leu Lys Lys Pro Gly Glu
        1               5                   10                  15


        Thr Val Lys Ile Ser Cys Lys Ala Ser Gly Tyr Thr Phe Thr Asn Ser
                        20                  25                  30


        Gly Met Asn Trp Val Lys Gln Ala Pro Gly Lys Gly Leu Lys Trp Met
                    35                  40                  45


        Gly Trp Ile Asn Thr Tyr Thr Gly Glu Pro Thr Tyr Ala Asp Asp Phe
                50                  55                  60
```

```
Lys Gly Arg Phe Ala Phe Ser Leu Glu Thr Ser Ala Ser Ser Ala Tyr
65                  70                  75                  80


Leu Gln Ile Ser Asn Leu Lys Asn Glu Asp Thr Ala Thr Tyr Phe Cys
                85                  90                  95


Ala Arg Arg Gly Phe Val Tyr Trp Gly Gln Gly Thr Leu Val Thr Val
            100                 105                 110


Ser Ala
```

<210> 53
<211> 360
<212> DNA
<213> Mus sp.

<220>
<221> CDS
<222> (1)..(360)

<400> 53

```
cag gtt cag ctc cag cag tct ggg gct gag ctg gca aga cct ggg act    48
Gln Val Gln Leu Gln Gln Ser Gly Ala Glu Leu Ala Arg Pro Gly Thr
1               5                   10                  15

tca gtg aag ttg tcc tgt aag gct tct ggc tac acc ttt act gac tac    96
Ser Val Lys Leu Ser Cys Lys Ala Ser Gly Tyr Thr Phe Thr Asp Tyr
            20                  25                  30

tgg atg cag tgg gta aaa cag agg cct gga cag ggt ctg gag tgg att   144
Trp Met Gln Trp Val Lys Gln Arg Pro Gly Gln Gly Leu Glu Trp Ile
        35                  40                  45

ggg act att tat cct gga gat ggt gat act ggg tac gct cag aag ttc   192
Gly Thr Ile Tyr Pro Gly Asp Gly Asp Thr Gly Tyr Ala Gln Lys Phe
    50                  55                  60

aag ggc aag gcc aca ttg act gcg gat aaa tcc tcc aaa aca gtc tac   240
Lys Gly Lys Ala Thr Leu Thr Ala Asp Lys Ser Ser Lys Thr Val Tyr
65                  70                  75                  80

atg cac ctc agc agt ttg gct tct gag gac tct gcg gtc tat tac tgt   288
Met His Leu Ser Ser Leu Ala Ser Glu Asp Ser Ala Val Tyr Tyr Cys
                85                  90                  95

gca aga ggg gat tac tac ggt agt aat tct ttg gac tat tgg ggt caa   336
Ala Arg Gly Asp Tyr Tyr Gly Ser Asn Ser Leu Asp Tyr Trp Gly Gln
            100                 105                 110

gga acc tca gtc acc gtc tcc tca                                    360
Gly Thr Ser Val Thr Val Ser Ser
            115                 120
```

<210> 54
<211> 120
<212> PRT
<213> Mus sp.

<400> 54

```
Gln Val Gln Leu Gln Gln Ser Gly Ala Glu Leu Ala Arg Pro Gly Thr
1               5                   10                  15

Ser Val Lys Leu Ser Cys Lys Ala Ser Gly Tyr Thr Phe Thr Asp Tyr
            20                  25                  30

Trp Met Gln Trp Val Lys Gln Arg Pro Gly Gln Gly Leu Glu Trp Ile
            35                  40                  45

Gly Thr Ile Tyr Pro Gly Asp Gly Asp Thr Gly Tyr Ala Gln Lys Phe
        50                  55                  60

Lys Gly Lys Ala Thr Leu Thr Ala Asp Lys Ser Ser Lys Thr Val Tyr
65                  70                  75                  80

Met His Leu Ser Ser Leu Ala Ser Glu Asp Ser Ala Val Tyr Tyr Cys
                85                  90                  95

Ala Arg Gly Asp Tyr Tyr Gly Ser Asn Ser Leu Asp Tyr Trp Gly Gln
            100                 105                 110

Gly Thr Ser Val Thr Val Ser Ser
        115                 120
```

<210> 55
<211> 357
<212> DNA
<213> Mus sp.

<220>
<221> CDS
<222> (1)..(357)

<400> 55

```
cag gtc cag tta cag caa tct gga cct gaa ctg gtg agg cct ggg gcc      48
Gln Val Gln Leu Gln Gln Ser Gly Pro Glu Leu Val Arg Pro Gly Ala
1                   5                   10                  15

tca gtg aag att tcc tgc aaa act tct ggc tac gca ttc agt ggc tcc      96
Ser Val Lys Ile Ser Cys Lys Thr Ser Gly Tyr Ala Phe Ser Gly Ser
                20                  25                  30

tgg atg aac tgg gtg aag cag agg cct gga cag ggt cta gag tgg att     144
Trp Met Asn Trp Val Lys Gln Arg Pro Gly Gln Gly Leu Glu Trp Ile
        35                  40                  45

gga cgg att tat ccg gga gat gga gat atc att tac aat ggg aat ttc     192
Gly Arg Ile Tyr Pro Gly Asp Gly Asp Ile Ile Tyr Asn Gly Asn Phe
        50                  55                  60

agg gac aag gtc aca ctg tct gca gac aaa tcc tcc aac aca gcc tac     240
Arg Asp Lys Val Thr Leu Ser Ala Asp Lys Ser Ser Asn Thr Ala Tyr
65                  70                  75                  80

atg cag ctc agc agc ctg acc tct gtg gac tct gcg gtc tat ttt tgt     288
Met Gln Leu Ser Ser Leu Thr Ser Val Asp Ser Ala Val Tyr Phe Cys
                85                  90                  95

tcg aga tgg ggg aca ttt acg ccg agt ttt gac tat tgg ggc caa ggc     336
Ser Arg Trp Gly Thr Phe Thr Pro Ser Phe Asp Tyr Trp Gly Gln Gly
                100                 105                 110

acc act ctc aca gtc tcc tca                                          357
Thr Thr Leu Thr Val Ser Ser
                115
```

<210> 56
<211> 119
<212> PRT
<213> Mus sp.

<400> 56

```
Gln Val Gln Leu Gln Gln Ser Gly Pro Glu Leu Val Arg Pro Gly Ala
1               5                   10                  15

Ser Val Lys Ile Ser Cys Lys Thr Ser Gly Tyr Ala Phe Ser Gly Ser
                20                  25                  30

Trp Met Asn Trp Val Lys Gln Arg Pro Gly Gln Gly Leu Glu Trp Ile
            35                  40                  45

Gly Arg Ile Tyr Pro Gly Asp Gly Asp Ile Ile Tyr Asn Gly Asn Phe
        50                  55                  60

Arg Asp Lys Val Thr Leu Ser Ala Asp Lys Ser Ser Asn Thr Ala Tyr
65                  70                  75                  80

Met Gln Leu Ser Ser Leu Thr Ser Val Asp Ser Ala Val Tyr Phe Cys
                85                  90                  95

Ser Arg Trp Gly Thr Phe Thr Pro Ser Phe Asp Tyr Trp Gly Gln Gly
            100                 105                 110

Thr Thr Leu Thr Val Ser Ser
            115
```

<210> 57
<211> 351
<212> DNA
<213> Mus sp.

<220>
<221> CDS
<222> (1)..(351)

<400> 57

```
gac gtg aag ctg gtg gag tct ggg gga ggc tta gtg aag cct gga ggg          48
Asp Val Lys Leu Val Glu Ser Gly Gly Gly Leu Val Lys Pro Gly Gly
1               5                   10                  15

tcc ctg aaa ctc tcc tgt gaa gcc tct gga ttc act ttc agt agc tat          96
Ser Leu Lys Leu Ser Cys Glu Ala Ser Gly Phe Thr Phe Ser Ser Tyr
                20                  25                  30

acc ctg tct tgg gtt cgc cag act ccg gag acg agg ctg gag tgg gtc         144
Thr Leu Ser Trp Val Arg Gln Thr Pro Glu Thr Arg Leu Glu Trp Val
            35                  40                  45

gca acc att agt att ggt ggt cgc tac acc tat tat cca gac agt gtg         192
Ala Thr Ile Ser Ile Gly Gly Arg Tyr Thr Tyr Tyr Pro Asp Ser Val
        50                  55                  60

gag ggc cga ttc acc atc tcc aga gac aat gcc aag aac acc ctg tac         240
Glu Gly Arg Phe Thr Ile Ser Arg Asp Asn Ala Lys Asn Thr Leu Tyr
65                  70                  75                  80

ctg caa atg aac agt ctg aag tct gag gac aca gcc atg tat tac tgt         288
Leu Gln Met Asn Ser Leu Lys Ser Glu Asp Thr Ala Met Tyr Tyr Cys
                85                  90                  95

aca aga gat ttt aat ggt tac tct gac ttc tgg ggc caa ggc acc act         336
Thr Arg Asp Phe Asn Gly Tyr Ser Asp Phe Trp Gly Gln Gly Thr Thr
                100                 105                 110

ctc aca gtc tcc tca                                                      351
Leu Thr Val Ser Ser
            115
```

<210> 58
<211> 117
<212> PRT
<213> Mus sp.

<400> 58

Asp Val Lys Leu Val Glu Ser Gly Gly Gly Leu Val Lys Pro Gly Gly
1               5              10                 15

Ser Leu Lys Leu Ser Cys Glu Ala Ser Gly Phe Thr Phe Ser Ser Tyr
            20                  25                  30

Thr Leu Ser Trp Val Arg Gln Thr Pro Glu Thr Arg Leu Glu Trp Val
            35                  40                  45

Ala Thr Ile Ser Ile Gly Gly Arg Tyr Thr Tyr Tyr Pro Asp Ser Val
        50                  55                  60

Glu Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Met Tyr Tyr Cys
                85                  90                  95

Thr Arg Asp Phe Asn Gly Tyr Ser Asp Phe Trp Gly Gln Gly Thr Thr
            100                 105                 110

Leu Thr Val Ser Ser
            115

<210> 59
<211> 360
<212> DNA
<213> Mus sp.

<220>
<221> CDS
<222> (1)..(360)

<400> 59

```
aat gta cag ctg gta gag tct ggg gga ggc tta gtg cag cct gga ggg      48
Asn Val Gln Leu Val Glu Ser Gly Gly Gly Leu Val Gln Pro Gly Gly
1               5                   10                  15

tcc cgg aaa ctc tcc tgt gca gcc tct gga ttc act ttc agt aac ttt      96
Ser Arg Lys Leu Ser Cys Ala Ala Ser Gly Phe Thr Phe Ser Asn Phe
            20                  25                  30

gga atg cac tgg gtt cgt cag gct cca gag aag ggt ctg gag tgg gtc     144
Gly Met His Trp Val Arg Gln Ala Pro Glu Lys Gly Leu Glu Trp Val
        35                  40                  45

gca tac att cgt agt ggc agt ggt acc atc tac tat tca gac aca gtg     192
Ala Tyr Ile Arg Ser Gly Ser Gly Thr Ile Tyr Tyr Ser Asp Thr Val
    50                  55                  60

aag ggc cga ttc acc atc tcc aga gac aat ccc aag aac acc ctg ttc     240
Lys Gly Arg Phe Thr Ile Ser Arg Asp Asn Pro Lys Asn Thr Leu Phe
65                  70                  75                  80

ctg caa atg acc agt cta agg tct gag gac acg gcc atg tat tac tgt     288
Leu Gln Met Thr Ser Leu Arg Ser Glu Asp Thr Ala Met Tyr Tyr Cys
                85                  90                  95

gca aga tcc tac tat gat ttc ggg gcc tgg ttt gct tac tgg ggc caa     336
Ala Arg Ser Tyr Tyr Asp Phe Gly Ala Trp Phe Ala Tyr Trp Gly Gln
            100                 105                 110

ggg act ctg gtc act gtc tct gca                                      360
Gly Thr Leu Val Thr Val Ser Ala
        115                 120
```

<210> 60
<211> 120
<212> PRT
<213> Mus sp.

<400> 60

```
    Asn Val Gln Leu Val Glu Ser Gly Gly Gly Leu Val Gln Pro Gly Gly
    1               5                   10                  15


    Ser Arg Lys Leu Ser Cys Ala Ala Ser Gly Phe Thr Phe Ser Asn Phe
```

```
                     20                      25                      30

     Gly Met His Trp Val Arg Gln Ala Pro Glu Lys Gly Leu Glu Trp Val
             35                      40                      45


     Ala Tyr Ile Arg Ser Gly Ser Gly Thr Ile Tyr Tyr Ser Asp Thr Val
             50                      55                      60


     Lys Gly Arg Phe Thr Ile Ser Arg Asp Asn Pro Lys Asn Thr Leu Phe
     65                      70                      75                      80


     Leu Gln Met Thr Ser Leu Arg Ser Glu Asp Thr Ala Met Tyr Tyr Cys
                     85                      90                      95


     Ala Arg Ser Tyr Tyr Asp Phe Gly Ala Trp Phe Ala Tyr Trp Gly Gln
                 100                     105                     110


     Gly Thr Leu Val Thr Val Ser Ala
                 115                     120
```

<210> 61
<211> 324
<212> DNA
<213> Homo sapiens

<220>
<221> CDS
<222> (1)..(324)

<400> 61

```
gat atc gta atg acc cag tcc cac ctg agt atg agt acc tcc ctg gga        48
Asp Ile Val Met Thr Gln Ser His Leu Ser Met Ser Thr Ser Leu Gly
1               5                   10                  15

gat cct gtg tca atc act tgc aag gcc tca cag gat gtg agc acc gtc        96
Asp Pro Val Ser Ile Thr Cys Lys Ala Ser Gln Asp Val Ser Thr Val
            20                  25                  30

gtt gct tgg tat cag cag aag ccc ggg caa tca ccc aga cgt ctc atc       144
Val Ala Trp Tyr Gln Gln Lys Pro Gly Gln Ser Pro Arg Arg Leu Ile
        35                  40                  45

tac tca gca tca tac cgt tac atc ggg gtg cct gac cga ttt act ggc       192
Tyr Ser Ala Ser Tyr Arg Tyr Ile Gly Val Pro Asp Arg Phe Thr Gly
        50                  55                  60

tct ggc gct ggc aca gat ttc acc ttt aca att agt tcc gtc cag gcc       240
Ser Gly Ala Gly Thr Asp Phe Thr Phe Thr Ile Ser Ser Val Gln Ala
65                  70                  75                  80

gaa gac ctg gcc gtg tac tac tgc cag cag cac tac agt ccc cca tac       288
Glu Asp Leu Ala Val Tyr Tyr Cys Gln Gln His Tyr Ser Pro Pro Tyr
                85                  90                  95

act ttc ggg gga ggg act aag ctc gaa atc aaa cgt                       324
Thr Phe Gly Gly Gly Thr Lys Leu Glu Ile Lys Arg
                        100                 105
```

<210> 62
<211> 108
<212> PRT
<213> Homo sapiens

<400> 62

EP 2 370 096 B1

```
        Asp Ile Val Met Thr Gln Ser His Leu Ser Met Ser Thr Ser Leu Gly
        1               5                   10                  15


        Asp Pro Val Ser Ile Thr Cys Lys Ala Ser Gln Asp Val Ser Thr Val
                    20                  25                  30


        Val Ala Trp Tyr Gln Gln Lys Pro Gly Gln Ser Pro Arg Arg Leu Ile
                35                  40                  45


        Tyr Ser Ala Ser Tyr Arg Tyr Ile Gly Val Pro Asp Arg Phe Thr Gly
            50                  55                  60


        Ser Gly Ala Gly Thr Asp Phe Thr Phe Thr Ile Ser Ser Val Gln Ala
        65                  70                  75                  80


        Glu Asp Leu Ala Val Tyr Tyr Cys Gln Gln His Tyr Ser Pro Pro Tyr
                        85                  90                  95


        Thr Phe Gly Gly Gly Thr Lys Leu Glu Ile Lys Arg
                    100                 105
```

<210> 63
<211> 324
<212> DNA
<213> Homo sapiens

<220>
<221> CDS
<222> (1)..(324)

<400> 63

```
    gac att gtt atg gct caa agc cat ctg tct atg agc aca tct ctg gga        48
    Asp Ile Val Met Ala Gln Ser His Leu Ser Met Ser Thr Ser Leu Gly
    1               5                   10                  15

    gat cct gtg tcc atc act tgc aaa gcc agt caa gac gtg tct aca gtt        96
    Asp Pro Val Ser Ile Thr Cys Lys Ala Ser Gln Asp Val Ser Thr Val
                20                  25                  30

    gtt gca tgg tat caa cag aag cca ggc cag tca ccc aga cgg ctc att       144
    Val Ala Trp Tyr Gln Gln Lys Pro Gly Gln Ser Pro Arg Arg Leu Ile
            35                  40                  45

    tac tca gct tct tac cga tac atc ggg gtc cct gac aga ttt aca ggt       192
    Tyr Ser Ala Ser Tyr Arg Tyr Ile Gly Val Pro Asp Arg Phe Thr Gly
            50                  55                  60
```

38

```
agt ggg gcc ggt act gac ttc act ttt act atc tca tcc gta caa gcc      240
Ser Gly Ala Gly Thr Asp Phe Thr Phe Thr Ile Ser Ser Val Gln Ala
65              70              75              80

gaa gac ctg gca gta tat tac tgc cag caa cat tat tcc cca ccc tac      288
Glu Asp Leu Ala Val Tyr Tyr Cys Gln Gln His Tyr Ser Pro Pro Tyr
                85              90              95

aca ttc ggc ggg ggt act aag ctg gaa att aaa cgt                      324
Thr Phe Gly Gly Gly Thr Lys Leu Glu Ile Lys Arg
            100             105
```

<210> 64
<211> 108
<212> PRT
<213> Homo sapiens

<400> 64

```
Asp Ile Val Met Ala Gln Ser His Leu Ser Met Ser Thr Ser Leu Gly
1               5                   10                  15

Asp Pro Val Ser Ile Thr Cys Lys Ala Ser Gln Asp Val Ser Thr Val
            20                  25                  30

Val Ala Trp Tyr Gln Gln Lys Pro Gly Gln Ser Pro Arg Arg Leu Ile
            35                  40                  45

Tyr Ser Ala Ser Tyr Arg Tyr Ile Gly Val Pro Asp Arg Phe Thr Gly
        50                  55                  60

Ser Gly Ala Gly Thr Asp Phe Thr Phe Thr Ile Ser Ser Val Gln Ala
65              70                  75                  80

Glu Asp Leu Ala Val Tyr Tyr Cys Gln Gln His Tyr Ser Pro Pro Tyr
                85                  90                  95

Thr Phe Gly Gly Gly Thr Lys Leu Glu Ile Lys Arg
            100                 105
```

<210> 65
<211> 360
<212> DNA
<213> Homo sapiens

<220>
<221> CDS
<222> (1)..(360)

<400> 65

```
cag gta cag ctc gtt cag tcc ggc gcc gag gta gct aag cct ggt act        48
Gln Val Gln Leu Val Gln Ser Gly Ala Glu Val Ala Lys Pro Gly Thr
1               5                   10                  15

tcc gta aaa ttg tcc tgt aag gct tcc ggg tac aca ttt aca gac tac        96
Ser Val Lys Leu Ser Cys Lys Ala Ser Gly Tyr Thr Phe Thr Asp Tyr
            20                  25                  30

tgg atg cag tgg gta aaa cag cgg cca ggt cag ggc ctg gag tgg att       144
Trp Met Gln Trp Val Lys Gln Arg Pro Gly Gln Gly Leu Glu Trp Ile
        35                  40                  45

gga aca ata tat ccc ggc gac ggc gac aca ggc tat gcc cag aag ttt       192
Gly Thr Ile Tyr Pro Gly Asp Gly Asp Thr Gly Tyr Ala Gln Lys Phe
    50                  55                  60

caa ggc aag gca acc ctt act gct gat aaa tct tcc aag act gtc tac       240
Gln Gly Lys Ala Thr Leu Thr Ala Asp Lys Ser Ser Lys Thr Val Tyr
65                  70                  75                  80

atg cat ctg tct tcc ttg gca tct gag gat agc gct gtc tat tac tgt       288
Met His Leu Ser Ser Leu Ala Ser Glu Asp Ser Ala Val Tyr Tyr Cys
                85                  90                  95

gct agg ggg gac tac tat ggg tca aat tcc ctg gat tac tgg ggc cag       336
Ala Arg Gly Asp Tyr Tyr Gly Ser Asn Ser Leu Asp Tyr Trp Gly Gln
            100                 105                 110

ggc acc agt gtc acc gtg agc agc                                       360
Gly Thr Ser Val Thr Val Ser Ser
        115                 120
```

<210> 66
<211> 120
<212> PRT
<213> Homo sapiens

<400> 66

```
Gln Val Gln Leu Val Gln Ser Gly Ala Glu Val Ala Lys Pro Gly Thr
1               5                   10                  15

Ser Val Lys Leu Ser Cys Lys Ala Ser Gly Tyr Thr Phe Thr Asp Tyr
            20                  25                  30

Trp Met Gln Trp Val Lys Gln Arg Pro Gly Gln Gly Leu Glu Trp Ile
            35                  40                  45

Gly Thr Ile Tyr Pro Gly Asp Gly Asp Thr Gly Tyr Ala Gln Lys Phe
        50                  55                  60

Gln Gly Lys Ala Thr Leu Thr Ala Asp Lys Ser Ser Lys Thr Val Tyr
65                  70                  75                  80

Met His Leu Ser Ser Leu Ala Ser Glu Asp Ser Ala Val Tyr Tyr Cys
                85                  90                  95

Ala Arg Gly Asp Tyr Tyr Gly Ser Asn Ser Leu Asp Tyr Trp Gly Gln
            100                 105                 110

Gly Thr Ser Val Thr Val Ser Ser
                    115                 120
```

<210> 67
<211> 324
<212> DNA
<213> Homo sapiens

<220>
<221> CDS
<222> (1)..(324)

<400> 67

41

```
gac acc gtg atg acc cag tcc ccc tcc acc atc tcc acc tct gtg ggc      48
Asp Thr Val Met Thr Gln Ser Pro Ser Thr Ile Ser Thr Ser Val Gly
1               5                   10                  15

gac cgg gtg tcc atc acc tgt aag gcc tcc cag gtg gtg ggc tcc gcc      96
Asp Arg Val Ser Ile Thr Cys Lys Ala Ser Gln Val Val Gly Ser Ala
            20                  25                  30

gtg gcc tgg tat cag cag aag cct ggc cag tcc cct aag ctg ctg atc     144
Val Ala Trp Tyr Gln Gln Lys Pro Gly Gln Ser Pro Lys Leu Leu Ile
        35                  40                  45

tac tgg gcc tcc acc cgg cat acc ggc gtg cct gac cgg ttc acc ggc     192
Tyr Trp Ala Ser Thr Arg His Thr Gly Val Pro Asp Arg Phe Thr Gly
        50                  55                  60

tcc ggc agc ggc acc gac ttc acc ctg acc atc tcc aac gtg cag tcc     240
Ser Gly Ser Gly Thr Asp Phe Thr Leu Thr Ile Ser Asn Val Gln Ser
65                  70                  75                  80

gac gac ctg gcc gac tac ttc tgc cag cag tac aac tcc tac cct tac     288
Asp Asp Leu Ala Asp Tyr Phe Cys Gln Gln Tyr Asn Ser Tyr Pro Tyr
                85                  90                  95

acc ttt ggc ggc gga aca aag ctg gag atc aag cgt                     324
Thr Phe Gly Gly Gly Thr Lys Leu Glu Ile Lys Arg
                100                 105
```

<210> 68
<211> 108
<212> PRT
<213> Homo sapiens

<400> 68

```
Asp Thr Val Met Thr Gln Ser Pro Ser Thr Ile Ser Thr Ser Val Gly
1               5                   10                  15


Asp Arg Val Ser Ile Thr Cys Lys Ala Ser Gln Val Val Gly Ser Ala
            20                  25                  30


Val Ala Trp Tyr Gln Gln Lys Pro Gly Gln Ser Pro Lys Leu Leu Ile
        35                  40                  45


Tyr Trp Ala Ser Thr Arg His Thr Gly Val Pro Asp Arg Phe Thr Gly
        50                  55                  60
```

EP 2 370 096 B1

```
        Ser Gly Ser Gly Thr Asp Phe Thr Leu Thr Ile Ser Asn Val Gln Ser
        65                  70                  75                  80


        Asp Asp Leu Ala Asp Tyr Phe Cys Gln Gln Tyr Asn Ser Tyr Pro Tyr
                        85                  90                  95


        Thr Phe Gly Gly Gly Thr Lys Leu Glu Ile Lys Arg
                    100                 105
```

<210> 69
<211> 324
<212> DNA
<213> Homo sapiens

<220>
<221> CDS
<222> (1)..(324)

```
<400>  69
gac acc gtg atg acc cag tcc ccc tcc tcc atc tcc acc tcc atc ggc    48
Asp Thr Val Met Thr Gln Ser Pro Ser Ser Ile Ser Thr Ser Ile Gly
1               5                   10                  15

gac cgg gtg tcc atc acc tgt aag gcc tcc cag gtg gtg ggc tcc gcc    96
Asp Arg Val Ser Ile Thr Cys Lys Ala Ser Gln Val Val Gly Ser Ala
                20                  25                  30

gtg gcc tgg tat cag cag aag cct ggc cag tcc cct aag ctg ctg atc   144
Val Ala Trp Tyr Gln Gln Lys Pro Gly Gln Ser Pro Lys Leu Leu Ile
        35                  40                  45

tac tgg gcc tcc acc cgg cat acc ggc gtg cct gcc cgg ttc acc ggc   192
Tyr Trp Ala Ser Thr Arg His Thr Gly Val Pro Ala Arg Phe Thr Gly
        50                  55                  60

tcc ggc agc ggc acc gac ttc acc ctg acc atc tcc aac gtg cag tcc   240
Ser Gly Ser Gly Thr Asp Phe Thr Leu Thr Ile Ser Asn Val Gln Ser
65                  70                  75                  80

gag gac ctg gcc gac tac ttc tgc cag cag tac aac tcc tac cct tac   288
Glu Asp Leu Ala Asp Tyr Phe Cys Gln Gln Tyr Asn Ser Tyr Pro Tyr
                85                  90                  95

acc ttt ggc ggc gga aca aag ctg gag atc aag cgt                    324
Thr Phe Gly Gly Gly Thr Lys Leu Glu Ile Lys Arg
                100                 105
```

<210> 70
<211> 108
<212> PRT
<213> Homo sapiens

<400> 70

43

```
Asp Thr Val Met Thr Gln Ser Pro Ser Ser Ile Ser Thr Ser Ile Gly
1               5                   10                  15

Asp Arg Val Ser Ile Thr Cys Lys Ala Ser Gln Val Val Gly Ser Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Gly Val Pro Ala Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Asn Ser Tyr Pro Tyr
                85                  90                  95

Thr Phe Gly Gly Gly Thr Lys Leu Glu Ile Lys Arg
            100                 105
```

<210> 71
<211> 351
<212> DNA
<213> Homo sapiens

<220>
<221> CDS
<222> (1)..(351)

<400> 71

```
gag gtg cag ctg gtg gag tct ggc ggc gga ctg gtg aag cct ggc ggc      48
Glu Val Gln Leu Val Glu Ser Gly Gly Gly Leu Val Lys Pro Gly Gly
1               5                   10                  15

tcc ctg agg ctg tcc tgt gag gcc tcc ggc ttc acc ttc tcc tcc tac      96
Ser Leu Arg Leu Ser Cys Glu Ala Ser Gly Phe Thr Phe Ser Ser Tyr
             20                  25                  30

acc ctg tcc tgg gtg agg cag acc cct ggc aag ggc ctg gag tgg gtg     144
Thr Leu Ser Trp Val Arg Gln Thr Pro Gly Lys Gly Leu Glu Trp Val
         35                  40                  45

gcc acc atc tcc atc ggc ggc agg tac acc tac tac cct gac tcc gtg     192
Ala Thr Ile Ser Ile Gly Gly Arg Tyr Thr Tyr Tyr Pro Asp Ser Val
     50                  55                  60

aag ggc cgg ttc acc atc tcc cgg gac aac gcc aag aac acc ctg tac     240
Lys Gly Arg Phe Thr Ile Ser Arg Asp Asn Ala Lys Asn Thr Leu Tyr
65                  70                  75                  80

ctg cag atg aac tcc ctg aag tcc gag gac acc gcc atg tac tac tgt     288
Leu Gln Met Asn Ser Leu Lys Ser Glu Asp Thr Ala Met Tyr Tyr Cys
                 85                  90                  95

acc cgg gac ttc aac ggc tac tcc gac ttc tgg ggc cag ggc acc aca     336
Thr Arg Asp Phe Asn Gly Tyr Ser Asp Phe Trp Gly Gln Gly Thr Thr
                100                 105                 110

ctg acc gtg tcc tcc                                                  351
Leu Thr Val Ser Ser
```

                                        115

<210> 72
<211> 117
<212> PRT
<213> Homo sapiens

<400> 72

```
Glu Val Gln Leu Val Glu Ser Gly Gly Gly Leu Val Lys Pro Gly Gly
1               5                   10                  15

Ser Leu Arg Leu Ser Cys Glu Ala Ser Gly Phe Thr Phe Ser Ser Tyr
            20                  25                  30

Thr Leu Ser Trp Val Arg Gln Thr Pro Gly Lys Gly Leu Glu Trp Val
            35                  40                  45

Ala Thr Ile Ser Ile Gly Gly Arg Tyr Thr Tyr Tyr Pro Asp Ser Val
        50                  55                  60

Lys Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Met Tyr Tyr Cys
                85                  90                  95

Thr Arg Asp Phe Asn Gly Tyr Ser Asp Phe Trp Gly Gln Gly Thr Thr
            100                 105                 110

Leu Thr Val Ser Ser
            115
```

<210> 73
<211> 36
<212> DNA
<213> Mus sp.

<400> 73
ggaggatcca tagacagatg ggggtgtcgt tttggc          36

<210> 74
<211> 32
<212> DNA
<213> Mus sp.

<400> 74
ggaggatccc ttgaccaggc atcctagagt ca          32

<210> 75
<211> 32
<212> DNA
<213> Mus sp.

<220>
<221> misc_feature
<222> (1)..(32)
<223> mixed bases are defined as follows: H=A+T+C, S=G+C, Y=C+T, K= G+T, M=A+C, R=A+G, W=A+T, V = A+C+G, N = A+C+G+T

<400> 75
cttccggaat tcsargtnma gctgsagsag tc          32


<210> 76
<211> 35
<212> DNA
<213> Mus sp.


<220>
<221> misc_feature
<222> (1)..(35)
<223> mixed bases are defined as follows: H=A+T+C, S=G+C, Y=C+T, K= G+T, M=A+C, R=A+G, W=A+T, V = A+C+G, N = A+C+G+T


<400> 76
cttccggaat tcsargtnma gctgsagsag tcwgg          35


<210> 77
<211> 31
<212> DNA
<213> Mus sp.


<220>
<221> misc_feature
<222> (1)..(31)
<223> mixed bases are defined as follows: H=A+T+C, S=G+C, Y=C+T, K= G+T, M=A+C, R=A+G, W=A+T, V = A+C+G, N = A+C+G+T


<400> 77
ggagctcgay attgtgmtsa cmcarwctmc a          31


<210> 78
<211> 46
<212> DNA
<213> Mus sp.


<400> 78
tatagagctc aagcttggat ggtgggaaga tggatacagt tggtgc          46


<210> 79
<211> 21
<212> DNA
<213> Mus sp.


<400> 79
atggagtcac agattcaggt c          21


<210> 80
<211> 32
<212> DNA
<213> Mus sp.


<400> 80
ttttgaattc cagtaacttc aggtgtccac tc          32